# EUROPÄISCHE PATENTANMELDUNG

(11) **EP 2 907 575 A1**
(43) Veröffentlichungstag der Anmeldung: **19.08.2015**
(21) Anmeldenummer: 14000534.9
(22) Anmeldetag: 14.02.2014
(51) Int. Cl.: B01L 3/02, B01L 7/00, G06F 3/0488, G01N 35/00

(54) **Laborgerät mit Benutzereingabefunktion und Verfahren zur Benutzereingabe bei einem Laborgerät**

(71) Anmelder: EPPENDORF AG, 22339 Hamburg (DE)
(72) Erfinder: Lüdicke, Sven, D-20099 Hamburg (DE); Poggenclaas, Martin, D-20255 Hamburg (DE); Roth, Stefan, D-22395 Hamburg (DE); Schicke, Kirsten, D-24568 Kaltenkirchen (DE)
(74) Vertreter: Ricker, Mathias

(57) **Zusammenfassung**

Die Erfindung bezieht sich auf ein Laborgerät, und ein Verfahren, zur gerätegesteuerten Behandlung mindestens einer Laborprobe, das Laborgerät aufweisend mindestens eine Behandlungseinrichtung, wobei die Behandlung unter Verwendung von mehreren Programmparametern vom Laborgerät gesteuert wird, eine Steuereinrichtung, eine Benutzerschnittstelleneinrichtung zur manuellen Eingabe von Daten durch einen Benutzer, und zur Anzeige von Informationen, die insbesondere von diesen Daten abhängig sein können, wobei die Benutzerschnittstelleneinrichtung ein Display aufweist, auf dem ein Anzeigebereich darstellbar ist, und wobei die Benutzerschnittstelleneinrichtung eine bewegungserfassende Sensoreinrichtung aufweist, die zur Erfassung mindestens einer Benutzerbewegung eingerichtet ist, die von einem Benutzer an dem Anzeigebereich durchführbar ist, und wobei die Steuereinrichtung zur Bereitstellung eines bewegungserfassenden Eingabemodus eingerichtet ist, um • in Abhängigkeit von der mindestens einen Benutzerbewegung den mindestens einen Programmparameter zu wählen und/oder dessen Wert festzulegen, und • in Abhängigkeit von der mindestens einen Benutzerbewegung in dem Anzeigebereich mindestens ein grafisches Skizzenelement anzuzeigen, dass die mindestens eine Benutzerbewegung repräsentiert.

## Beschreibung

Die Erfindung bezieht sich auf Laborgerät mit Benutzereingabefunktion und Verfahren zur Benutzereingabe bei einem Laborgerät.

Solche Laborgeräte werden verwendet, um in chemischen, biologischen, biochemischen, medizinischen oder forensischen Laboratorien Laborproben, insbesondere flüssige Laborproben, mit hoher Effizienz zu bearbeiten. Solche Laborgeräte automatisieren Behandlungsschritte zumindest teilweise, die sonst manuell durchgeführt werden müssten, und steigern auf diese Weise die Geschwindigkeit, Präzision und Zuverlässigkeit dieser Behandlungen. Eine Behandlung von zumeist flüssigen Laborproben kann darauf gerichtet sein, diese Laborproben, insbesondere deren Zusammensetzung, physikalisch, chemisch, biochemisch oder auf andere Weise zu ändern oder zu untersuchen.

Eine Behandlung einer flüssigen Laborprobe kann darauf gerichtet sein, diese Probe physikalisch, chemisch, biochemisch oder auf andere Weise in ihrer Zusammensetzung zu ändern. Durch eine Behandlung der Probe kann eine Probe z.B. geteilt oder verdünnt werden. Die Inhaltsstoffe einer Probe können analysiert werden oder es können, z.B. durch eine chemische Reaktion, neue Inhaltsstoffe hergestellt werden, insbesondere unter Verwendung der Probe. Insbesondere im Zusammenhang mit der Bearbeitung und Analyse von DNA oder RNA oder deren Bestandteilen sind Laborgeräte hilfreich, um eine Fülle von Informationen innerhalb einer geeigneten Zeitspanne zu gewinnen oder viele solcher Proben zu analysieren.

Die genannten Laborgeräte weisen eine oder mehrere Behandlungseinrichtung(en) zur gerätegesteuerten Behandlung der mindestens einen Laborprobe auf. Sie weisen oft eine Programmsteuerung auf, mittels der ein Benutzer des Laborgeräts die durchzuführende Behandlung durch Einstellen der gewünschten Programmparameter festlegen kann. Die Einstellung der Programmparameter erfolgt über eine Bedieneinheit des Laborgeräts, welche die Ein- und Ausgabe von Informationen, insbesondere von Werten der Programmparameter ermöglicht.

Die Programmierung von bekannten Laborgeräten wurde oft als unkomfortabel und wenig anwenderfreundlich beschrieben. Mitunter mussten vom Anwender umfangreiche numerische Eingaben mittels einer Tastatur oder anderer Eingabemittel getätigt werden, bevor alle für die automatische Abarbeitung einer Probenbehandlung notwendig vom Benutzer einzugebenden Programmparameter eingegeben worden waren und die Probenbehandlung gestartet werden konnte.

Aufgabe der vorliegenden Erfindung ist es, ein verbessertes Laborgerät mit Benutzereingabefunktion und ein verbessertes Verfahren zur Benutzereingabe bei einem Laborgerät bereitzustellen, mit dem sich die Produktivität in einem Labor verbessern lässt.

Die Erfindung löst diese Aufgabe insbesondere durch das Laborgerät gemäß Anspruch 1 und das Verfahren nach Anspruch 13. Bevorzugte Ausgestaltungen sind insbesondere Gegenstände der Unteransprüche.
Das erfindungsgemäße Laborgerät zur gerätegesteuerten Behandlung mindestens einer Laborprobe, weist auf: - mindestens eine Behandlungseinrichtung zur programmgesteuerten Behandlung der mindestens einen Laborprobe, wobei die Behandlung unter Verwendung von mehreren Programmparametern, die zumindest teilweise als Benutzerparameter vom Benutzer bestimmt werden, vom Laborgerät gesteuert wird, - eine Steuereinrichtung, aufweisend mindestens eine Prozessoreinrichtung zur Datenverarbeitung, wobei diese Datenverarbeitung die Ausführung eines Steuerprogramms zum Steuern des Laborgeräts beinhaltet, und aufweisend mindestens einer Speichereinrichtung zum Speichern von Daten, insbesondere dem Steuerprogramm und den Programmparametern, - eine Benutzerschnittstelleneinrichtung zur manuellen Eingabe von Daten durch einen Benutzer, und zur Anzeige von Informationen, die insbesondere von diesen Daten abhängig sein können, wobei die Benutzerschnittstelleneinrichtung ein Display aufweist, auf dem ein Anzeigebereich darstellbar ist, und wobei die Benutzerschnittstelleneinrichtung eine bewegungserfassende Sensoreinrichtung aufweist, die zur Erfassung mindestens einer Benutzerbewegung eingerichtet ist, die von einem Benutzer an dem Anzeigebereich durchführbar ist, und wobei die Steuereinrichtung zur Bereitstellung eines bewegungserfassenden Eingabemodus eingerichtet ist, um • in Abhängigkeit von der mindestens einen Benutzerbewegung den mindestens einen Programmparameter zu wählen und/oder dessen Wert festzulegen, und • in Abhängigkeit von der mindestens einen Benutzerbewegung in dem Anzeigebereich mindestens ein grafisches Skizzenelement anzuzeigen, dass die mindestens eine Benutzerbewegung repräsentiert.

Ein grafisches Skizzenelement kann ein kontinuierliches grafisches Objekt sein, z.B. ein ununterbrochener Pfad, insbesondere eine durchgehende, teilweise oder vollständig gekrümmte oder nicht gekrümmte -also gerade- Linie, bzw. eine Kurve mit oder ohne lineare Abschnitte, oder kann ein diskontinuierliches grafisches Objekt sein, z.B. ein unterbrochener Pfad, insbesondere eine Abfolge aus zwei oder mehr oder vielen Punkten, wobei jeder Punkt durch ein diskretes grafisches Symbol repräsentiert sein kann, z.B. ein Quadrat, Rechteck, Kreis, Kreuz, etc.

Das Laborgerät, insbesondere das Steuerprogramm, ist dazu eingerichtet, die Behandlung mindestens einer Laborprobe in Abhängigkeit von dem mindestens einen Programmparameter durchzuführen, der durch die Benutzerbewegung gewählt wird und/oder dessen Wert durch diese Benutzerbewegung bestimmt wird. Die Benutzerbewegung wird auf diese Weise als Eingabetätigkeit wirksam, ähnlich wie das Drücken einer Taste oder die Bewegung einer Computermaus oder eines Joysticks. Die grafische Repräsentation der Benutzerbewegung im Anzeigebereich des Displays erfolgt vorzugsweise im wesentlichen ohne eine für den Benutzer wahrnehmbare Zeitverzögerung, so dass der Benutzer ein visuelles Feedback und somit eine visuelle Kontrollmöglichkeit seiner Benutzerbewegung erhält. Auf diese Weise wird die Benutzung des Laborgeräts intuitiv. Insbesondere lassen sich Programmparameter auf diese Weise intuitiv am Laborgerät eingeben. Die Bedienung wird so signifikant erleichtert, insbesondere lässt sich dank der intuitiven Steuerung ein effizienterer Workflow im Labor erzielen.

Diese bewegungsgesteuerte Eingabe ist vorzugsweise möglich, wenn die Benutzerschnittstelleneinrichtung durch ein Ereignis in einen Eingabemodus versetzt worden ist, und ist vorzugsweise nicht möglich, wenn die Benutzerschnittstelleneinrichtung nicht in einen Eingabemodus versetzt worden ist. Dadurch wird verhindert, dass ungewollte Eingaben am Anzeigebereich durchgeführt werden. Das genannte Ereignis kann vom Steuerprogramm festgelegt sein, indem z.B. das Steuerprogramm als Software-Betriebssystem des Laborgeräts arbeitet, das ein Programm, insbesondere ein Unterprogramm oder ein Programmmodul oder ein Methodenprogramm ausführt. Bei der Ausführung dieses Programms werden die den Eingabemodus startenden Ereignisse ausgelöst, wenn eine Benutzereingabe durch Benutzerbewegung erfolgen soll. Ebenso programmgesteuert kann der Eingabemodus wieder beendet werden. Es ist aber auch möglich und bevorzugt, dass der Benutzer das Starten und/oder Beenden des Eingabemodus über Eingaben steuert, die er über die Benutzerschnittstelle vornimmt. Ferner kann das Starten und/oder Beenden des Eingabemodus auch über eine Fernsteuerung erfolgen, die mittels einer Datenschnittstelle realisiert sein kann. Diese kann zur drahtgebundenen oder drahtlosen Datenkommunikation ausgebildet sein, unidirektional oder bidirektional.

Dabei ist auch bevorzugt, dass -insbesondere gleichzeitig während des Eingabemodusauch weitere Benutzereingaben an der Benutzerschnittstelle erfolgen können, z.B. durch Betätigen von Hardware-Eingabemitteln, z.B. von Tasten, Eingabefeldern, Bedienrädern oder Bedienwippen, oder von virtuellen Eingabemitteln, z.B. auf einem Anzeigebereich eines berührungsempfindlichen Display (Touchscreen) anzeigbaren Eingabeflächen. Es ist möglich und bevorzugt, dass mittels der Benutzerbewegung zunächst ein Programmparameter ausgewählt wird, vorzugsweise aus dieser oder einer anderen -insbesondere unmittelbar oder zeitlich versetzt nachfolgenden- Benutzerbewegung der Wert dieses Programmparameters festgelegt wird, und dass vorzugsweise anschließend dem Benutzer die Möglichkeit angeboten wird, den mittels der Benutzerbewegung eingegebenen Wert zu ändern, insbesondere nachzustellen. Dies kann durch eine erneute Bewegungssteuerung erfolgen, und/oder durch Betätigung von Hardware-Eingabemitteln. Die genannten Funktionalitäten des Laborgeräts sind vorzugsweise computerimplementiert. Sie lassen sich insbesondere dadurch realisieren, dass das Steuerprogramm, insbesondere die von diesem vorzugsweise verwendeten Unterprogramme, Programmmodule oder Methodenprogramme, entsprechend ausgebildet sind, insbesondere programmiert sind, um die Steuerung des Laborgeräts zur Durchführung der jeweiligen Funktionalität zu ermöglichen. Dies gilt auch für die in dieser Patentanmeldung weiter beschriebenen bevorzugten Funktionen und Fähigkeiten des erfindungsgemäßen Laborgeräts, falls technisch möglich und falls nicht anders beschrieben.

Die bewegungserfassende Sensoreinrichtung ist vorzugsweise die berührungssensitive Schicht des Displays, das vorzugsweise als Touchscreen ausgeführt ist. Eine Berührung kann dabei mittels eines Fingers des Benutzers erfolgen oder mittels einer vom Benutzer geführten Eingabehilfe, z.B. eines Eingabestiftes. Die bewegungserfassende Sensoreinrichtung kann auch zur berührungslosen Erfassung der Benutzerbewegung eingerichtet sein. Dazu kann die Sensoreinrichtung insbesondere mindestens eine Kamera aufweisen, insbesondere Infrarotkamera, mittels welcher Aufnahmen von am Anzeigenbereich durchgeführten Benutzerbewegungen erstellbar sind. Diese Aufnahmen sind insbesondere von der Benutzerschnittstellensteuerung insbesondere mittels Bildverarbeitungsprogrammen auswertbar, um die Benutzerbewegung zu erfassen. Bevorzugte Techniken zur Bewegungserfassung, insbesondere Touchscreens und insbesondere berührungslos arbeitende Sensoreinrichtungen, bieten den Vorteil einer reibungsarmen oder reibungsfreien und damit besonders ergonomischen Interaktion des Benutzers.

Eine Benutzerschnittstelleneinrichtung, insbesondere ein Touchscreen, stellt eine Eingabeschnittstelle und eine Ausgabeschnittstelle zwischen dem Laborgerät und einem Benutzer bereit. Die Steuereinrichtung des Laborgeräts kann die Steuereinrichtung zur Steuerung der Benutzerschnittstelleneinrichtung beinhalten, die auch als Benutzerschnittstellensteuerung bezeichnet wird. In diesem Fall werden sowohl die Funktionen der Steuerung der mindestens einen Behandlungseinrichtung als auch die Benutzersteuerung an der Benutzerschnittstelleneinrichtung von der Steuereinrichtung durchgeführt, insbesondere mittels einer einzigen Datenverarbeitungseinrichtung, insbesondere Mikroprozessoreinrichtung. Die Steuereinrichtung des Laborgeräts und die Benutzerschnittstellensteuerung können aber auch als separate Bauteile ausgeführt sein, die insbesondere jeweils eine eigene Datenverarbeitungseinrichtung, insbesondere Mikroprozessor, aufweisen.

Die Steuereinrichtung, und/oder die Benutzerschnittstellensteuerung ist vorzugsweise dazu eingerichtet, elektrische Signale von einem Touchscreen zu empfangen oder an diesen zu senden. Das Display, insbesondere der Touchscreen, zeigt visuelle Ausgaben an den Benutzer an. Die visuellen Ausgaben können verschiedene Ausgabeelemente aufweisen, beinhaltend z.B. Graphik, insbesondere Rastergrafik oder Vektorgrafik, Text, Icons, Video und jede Kombination davon.
Ein Touchscreen kann insbesondere die bewegungserfassende Sensoreinrichtung aufweisen. Ein Touchscreen hat eine berührungsempfindliche Oberfläche, einen Sensor oder eine Gruppe an Sensoren, die Eingaben von einem Benutzer basierend auf haptischen und/oder taktilem Kontakt akzeptiert. Der Touchscreen mit der Benutzerschnittstellensteuerung erfasst insbesondere den Kontakt, insbesondere die Bewegung oder Unterbrechung des Kontaktes, auf dem Touchscreen und interpretiert insbesondere den erfassten Kontakt als eine Interaktion des Benutzers mit Eingabebereichen, die insbesondere in dem Anzeigebereich angeordnet und insbesondere für den Benutzer erkennbar sein können. Vorzugsweise korrespondiert ein Kontaktpunkt zwischen einem Touchscreen und dem Benutzer mit der Fingerspitze des Benutzers.

Der Touchscreen kann insbesondere LCD (liquid crystal display) Technologie oder LPD (light emitting polymer display) Technologie verwenden. Der Touchscreen und die Benutzerschnittstellensteuerung können Kontakt und Bewegung oder Unterbrechung davon erfassen. Die dazu vorzugsweise eingesetzte Sensoreinrichtung kann eine kapazitive, resistive, infrarot oder akustische-oberflächenwellen Technologie verwenden, sowie andere Nahbereichsensorfelder. Der Touchscreen kann z.B. eine Auflösung von vorzugsweise größer als 60 dpi oder 100 dpi haben, insbesondere zwischen 60 dpi und 350 dpi, oder zwischen 100 dpi und 250 dpi. Der Benutzer kann den Touchscreen unter Verwendung eines geeigneten Objektes oder Körperteils, wie z. B. einen Stift, einen Finger, kontaktieren. Die Benutzerschnittstellensteuerung kann insbesondere dazu eingerichtet sein, die groben, fingerbasierten Eingaben in eine präzise Position bzw. Bewegung eines auf dem Anzeigebereichs dargestellten Objektes zu übertragen, z.B. Cursor, Mauszeiger, Icon, Graph, etc..

Eine Benutzerschnittstelleneinrichtung kann ein Bauteil des Laborgeräts sein, das ein fester Bestandteil des Laborgeräts sein kann oder, in einer alternativen Ausführungsform, das separat von dem Laborgerät betrieben werden kann, in dem es über eine Schnittstelleneinrichtung, insbesondere über elektrische Kontakte oder drahtlos mit der Steuereinrichtung des Laborgeräts kommuniziert. Eine insbesondere auch unabhängig vom Laborgerät betreibbare Benutzerschnittstelleneinrichtung kann insbesondere eine eigene Datenverarbeitungseinrichtung aufweisen, und insbesondere eine eigene Energiequelle, z.B. Batterie. Die Benutzerschnittstelleneinrichtung kann insbesondere eine mobile Computervorrichtung sein, insbesondere ein Tabletcomputer oder ein Smart-Phone. Diese mobile Computervorrichtung ist vorzugsweise zur Kommunikation mit dem Laborgerät eingerichtet, z.B. mittels geeigneter Software und/oder Schnittstelleneinrichtung. Diese mobile Computervorrichtung kann ein separat kommerziell erhältlicher Tabletcomputer oder separat kommerziell erhältliches Smart-Phone sein oder kann ein separat nicht kommerziell erhältlicher Tabletcomputer oder nicht kommerziell erhältliches Smart-Phone sein. Die mobile Computervorrichtung kann dazu eingerichtet sein, für mehr als ein Laborgerät, insbesondere für mehr als einen behandlungsspezifischen Typ von Laborgerät als Benutzerschnittstelleneinrichtung verwendbar zu sein.

Es ist möglich und bevorzugt, dass der mindestens eine Programmparameter durch die Benutzerbewegung gewählt wird. Das Laborgerät erkennt insbesondere aus der Benutzerbewegung, welcher Programmparameter vom Benutzer durch die Benutzerbewegung ausgewählt wird. Zur Erläuterung sei auf die Ausführungsbeispiele verwiesen: bei einer als senkrechter Strichgeste durchgeführten Benutzerbewegung erkennt ein als Thermocycler ausgeführtes erfindungsgemäßes Laborgerät vorzugsweise, dass der Benutzer den Programmparameter "Zykluszeit" gewählt hat. Bei einer als horizontaler Strichgeste durchgeführten Benutzerbewegung erkennt das Laborgerät vorzugsweise, dass der Benutzer den Programmparameter "Zyklustemperatur" gewählt hat. Vorzugsweise dieselbe Eingabe, kann in der Weise vom Laborgerät interpretiert werden, dass sie einen Wert für den gewählten Programmparameter festlegt. Im Beispiel definiert z.B. die horizontale Position der vertikalen Benutzerbewegung am Anzeigebereich den Wert des Programmparameters "Zykluszeit", die vertikale Position der horizontalen Benutzerbewegung am Anzeigebereich bestimmt den Wert des Programmparameters "Zyklustemperatur".

Alternativ zu einer Benutzerbewegung, die sowohl die Auswahl des Programmparameters und gleichzeitig auch den Wert des Programmparameters festlegt, kann auch eine erste Eingabe des Benutzers, insbesondere mittels einer Benutzerbewegung, verwendet werden, um die Auswahl des Programmparameters durchzuführen. Eine zweite Eingabe, insbesondere eine zweite Benutzerbewegung, die insbesondere unmittelbar oder im zeitlichen Abstand nach der ersten Eingabe erfasst werden kann, kann dann den Wert des gewählten Programmparameters festlegen.

Die Benutzerbewegung ist vorzugsweise eine Geste. Eine Geste ist insbesondere dadurch definierbar, dass sie als Bewegung eines Körpers verstanden wird, welche Information enthält. Eine Geste kann aber auch so definierbar sein, dass darunter z.B. eine bestimmte Körperkonfiguration, z.B. Handhaltung, verstanden wird, welche die Information beinhaltet. Eine Geste kann eine Einzelobjekt-Geste sein oder eine Mehrfachobjekt-Geste. Eine Einzelobjekt-Geste berücksichtigt insbesondere nur die Bewegung eines einzigen Referenzpunktes, z.B. den Kontaktpunkt der Fingerspitze eines Benutzers am Anzeigebereich eines Touchscreens oder die Bewegung einer einzelnen Hand im Detektionsbereich einer berührungslos arbeitenden Sensoreinrichtung am Anzeigenbereich. Eine Mehrfachobjekt-Geste kann mehrere, vorzugsweise zwei, simultan bewegte Referenzpunkte auswerten, z.B. die Kontaktpunkte mehrerer Fingerspitzen oder zweier Hände.

Eine Geste kann eine Bewegung des Referenzpunktes entlang einer vorbestimmten Bewegungsbahn sein, die insbesondere in ihrer Richtung und/oder in ihrer Länge bestimmt sein kann. Vorzugsweise werden weniger als 5, 10 oder 15, leicht unterscheidbare Gesten vorgesehen. Vorzugsweise sind dies geradlinige Gesten, insbesondere horizontale oder vertikale Gesten, oder schräge geradlinige Gesten, insbesondere ca. 45° Gesten, wobei die Länge der Bewegung vorzugsweise nicht ausgewertet wird. Vorzugsweise sind diese Gesten auch Kombinationen aus verschiedenen geradlinigen Abschnitten, zum Beispiel Winkelgesten, die z.B. einen vertikalen und einen horizontalen Abschnitt aufweisen können, die insbesondere noch in ihrer relativen Ausrichtung unterscheidbar sein können. Vorzugsweise sind diese Gesten auch geschwungene Bewegungsbahnen, z.B. eine kreisförmig, kurvenförmig, acht-förmig, und dergleichen.

Vorzugsweise ist die Steuereinrichtung dazu eingerichtet, im Anzeigebereich des Displays Informationen grafisch anzuzeigen, insbesondere Informationen über mindestens einen Programmparameter.

In einem bevorzugten Eingabemodus werden entlang einer ersten linearen Achse mögliche Werte mindestens eines ersten Programmparameters, z.B. eines Zeitwerts, angezeigt. Vorzugsweise ist die Steuereinrichtung dazu eingerichtet, in diesem Eingabemodus eine Benutzerbewegung senkrecht zu der einen ersten linearen Achse zu erfassen, um den mit dieser Achse assoziierten Programmparameters auszuwählen und/oder dessen Wert zu bestimmen. Vorzugsweise ist die Steuereinrichtung dazu eingerichtet, in diesem Eingabemodus eine Benutzerbewegung senkrecht zu der einen ersten linearen Achse zu erfassen, um den Wert des mit dieser Achse assoziierten Programmparameters auszuwählen. Dazu kann insbesondere eine Position der linearen, senkrechten Benutzerbewegung entlang der ersten linearen Achse erfasst werden, wobei diese Position für diesen Wert charakteristisch ist. Es ist auch möglich, dass mehrere Benutzerbewegungen zeitlich aufeinanderfolgend, insbesondere in vorbestimmten Zeitabständen aufeinanderfolgend, z.B. in Zeitabständen von 0,0 bis 3,0 Sekunden, oder gleichzeitig erfasst werden, um z.B. mehrere horizontale Positionen entlang dieser Achse zu erfassen. In diesem bevorzugten Eingabemodus werden vorzugsweise entlang einer zweiten linearen Achse mögliche Werte mindestens eines zweiten Programmparameters, z.B. einer Temperatur, angezeigt. Vorzugsweise ist die Steuereinrichtung dazu eingerichtet, in diesem Eingabemodus eine Benutzerbewegung senkrecht zu der einen zweiten linearen Achse zu erfassen, um den mit dieser Achse assoziierten Programmparameter auszuwählen und/oder dessen Wert zu bestimmten. Vorzugsweise ist die Steuereinrichtung dazu eingerichtet, in diesem Eingabemodus eine Benutzerbewegung senkrecht zu der einen zweiten linearen Achse zu erfassen, um den Wert des mit dieser Achse assoziierten Programmparameters auszuwählen. Dazu kann insbesondere eine Position der linearen, senkrechten Benutzerbewegung entlang der zweiten linearen Achse erfasst werden, wobei diese Position für diesen Wert charakteristisch ist. Es ist auch möglich, dass mehrere Benutzerbewegungen zeitlich aufeinanderfolgend oder gleichzeitig erfasst werden, um z.B. mehrere horizontale Positionen entlang dieser Achse zu erfassen.

Es ist möglich und bevorzugt, dass die Steuereinrichtung dazu eingerichtet ist, dass die Eingabe eines Wertes durch die Erfassung der Position der Benutzerbewegung senkrecht zur linearen Achse mit einer kontinuierlichen Ortsauflösung erfolgt, die z.B. technisch durch die Ortsauflösung der Sensoreinrichtung begrenzt sein kann. Es ist aber auch möglich und bevorzugt, dass die Steuereinrichtung dazu eingerichtet ist, die Eingabe eines Wertes durch die Erfassung der Position der Benutzerbewegung senkrecht zur linearen Achse mit einer verringerten Ortsauflösung, insbesondere inkrementell, durchzuführen. Dazu kann die Steuereinrichtung die Werte in vorbestimmten Abständen, bezeichnet als Inkremente, erfassen, welche die Auflösung der Erfassung der Werte beschränken können. Die verringerte Ortsauflösung kann insbesondere unter der Ortsauflösung der Sensoreinrichtung liegen, insbesondere wenn dies je nach Anwendungsfall sinnvoll ist. Bei chemischen Reaktionen, z.B. einer PCR, kann eine Ortsauflösung der Darstellung sinnvoll sein, die einer Zeitauflösung von 10 Sekunden bei der Definition eines PCR-Zyklus entspricht oder einer Temperaturauflösung von 0,1 °C entspricht.

In dem bevorzugten Eingabemodus sind vorzugsweise die erste und zweite Achse senkrecht zueinander ausgerichtet. Vorzugsweise ist das Display im Wesentlichen rechteckförmig mit zwei im Wesentlichen horizontal verlaufenden Rändern und zwei im Wesentlichen senkrecht zu den horizontalen Rändern verlaufenden Rändern. Dann verläuft die erste Achse vorzugsweise horizontal, und die zweite Achse verläuft vorzugsweise senkrecht zur Horizontalen, z.B. vertikal. In einem solchen Display lassen sich besonders effizient Prozessabläufe darstellen, bei denen sich ein Prozessparameter, z.B. eine Temperatur, gegenüber einem Ablaufparameter, z.B. repräsentierend einen Zeitwert oder Position in einer Reihenfolge, darstellen lässt. Typischerweise kann der Ablaufparameter horizontal, vorzugsweise parallel der x-Achse eines kartesischen Koordinatensystems, und der Prozessparameter senkrecht dazu, parallel der y-Achse des Koordinatensystems aufgetragen werden.

Vorzugsweise ist die Steuereinrichtung dazu eingerichtet, durch die Benutzerbewegung Paare von Programmparametern oder Paare von Werten eines Programmparameters oder von zwei Programmparametern zu erfassen. Eine Vielzahl solcher Paare können durch Punkte oder eine Kurve grafisch dargestellt werden, die an zueinander senkrechten linearen Achsen gegeneinander aufgetragen werden, zum Beispiel in einem kartesischen Koordinatensystem. Die Steuereinrichtung kann dazu eingerichtet sein, ein solches Koordinatensystem im Anzeigebereich darzustellen. Die Steuereinrichtung kann dazu eingerichtet sein, eine Benutzerbewegung, die am Anzeigebereich durchgeführt wird und die in diesem Koordinatensystem als kurvenförmiges grafisches Skizzenelement dargestellt wird, auszuwerten, um an diesem Koordinatensystem Paare von Programmparametern oder von Paare von Werten eines Programmparameters oder von zwei Programmparametern zu erfassen. Vorzugsweise ist die Steuereinrichtung dazu ausgebildet, dass der Benutzer einzelne Kurvenabschnitte nachträglich ändern kann und somit Paare von Programmparametern oder von Paare von Werten eines Programmparameters oder von zwei Programmparametern nachträglich ändern kann, indem die Eingaben aus weiteren Eingabemitteln der Benutzerschnittstelleneinrichtung erfasst werden oder indem eine weitere Benutzerbewegung am Anzeigebereich ausgewertet wird. Eine weitere Benutzerbewegung kann z.B. einen Abschnitt der die erste Benutzerbewegung repräsentierenden Kurve im Anzeigebereich berühren und dann insbesondere in einen anderen Anzeigebereich verschieben oder übertragen, insbesondere kontinuierlich oder inkrementell. Auf diese Weise kann insbesondere eine Prozessplanung bei einem automatisierten PCR Prozess intuitiv erfolgen, wie dies anhand der Figuren noch erläutert wird.

Vorzugsweise ist die Steuereinrichtung dazu eingerichtet, im Anzeigenbereich mehrere grafische Objekte darzustellen und eine im wesentlichen kreisförmige Benutzerbewegung, die mindestens eines -vorzugsweise mehrere- dieser grafischen Objekte berührt und/oder umrahmt, als Auswahlbewegung zu verwenden. Insbesondere ist es möglich, dass jedem grafischen Objekt ein Programmparameter zugeordnet ist und dass die Auswahlbewegung dazu führt, dass mindestens ein Programmparameter, vorzugsweise mehrere Programmparameter ausgewählt werden. Z.B. kann die Steuereinrichtung im Anzeigebereich mehrere Teilbereiche grafisch darstellen, wobei jeder Teilbereich für einen Programmparameter steht.

Ein erster Teilbereich kann z.B. im Rahmen der Planung eines PCR-Prozesses für den Prozessparameter "erste Temperatur" stehen, ein zweiter Teilbereich kann für den Prozessparameter "zweite Temperatur" stehen, ein dritter Teilbereich kann für den Prozessparameter "dritte Temperatur" stehen, wobei diese Teilbereiche z.B. horizontal nebeneinander dargestellt sein können. Eine Auswahlbewegung, deren Repräsentation als grafisches Skizzenelement mindestens einen, z.B. einen, zwei oder drei dieser Teilbereiche berührt, kann entsprechend mindestens einen, insbesondere einen, zwei oder drei der genannten Programmparameter auswählen; die Steuereinrichtung kann dazu ausgebildet sein, eine andere Eingabe des Benutzers zu erfassen, durch welche die mittels der Auswahlbewegung gewählten -oder nachträglich noch zu wählenden- Programmparameter, nämlich Temperaturen, einem Temperaturzyklus zugeordnet werden, der dann z.B. gemäß einer ebenfalls noch einzugebenden Anzahl von Wiederholungen wiederholt werden soll. Auf diese Weise wird die Prozessplanung mittels Auswahlbewegung erleichtert.

Es ist auch möglich und bevorzugt, dass - insbesondere bei der Ausführung des Laborgeräts als Pipettierautomat-, im Anzeigebereich ein Arbeitsbereich des Laborgeräts grafisch dargestellt wird, in dem mehrere Arbeitsstationen -vorzugsweise rasterförmig- angeordnet sind. Ein Laborgerät mit mehreren Arbeitsstationen kann z.B. mehrere Behandlungseinrichtungen aufweisen, die in einem automatisierten Prozess in einer bestimmten Reihenfolge zum Einsatz kommen. Bei einem Pipettierautomaten werden z.B. Proben und Labware, insbesondere Einzel- oder Mehrfach-Probengefäße, Pipettenspitzen, Flüssigkeitsvorräte, Werkzeuge, an vorbestimmten Arbeitsstationen des Arbeitsbereichs gelagert. An anderen Arbeitsstationen werden Proben pipettiert, temperaturbehandelt, insbesondere bei einer PCR zyklisch temperaturbehandelt, magnetisch behandelt, gemischt, bestrahlt etc. Durch die grafische Darstellung des Arbeitsbereichs und die erfindungsgemäße grafisch unterstützte Auswahl von Programmparametern kann z.B. in intuitiver Weise ein Prozess an mehreren Arbeitsstationen geplant werden. Ein erster im Anzeigenbereich dargestellter Teilbereich repräsentiert dann z.B. eine erste Arbeitsstation, ein zweiter Teilbereich repräsentiert eine zweite Arbeitsstation, ein dritter Teilbereich repräsentiert eine dritte Arbeitsstation, ein n-ter Teilbereich repräsentiert eine n-te Arbeitsstation (n=1...N, N eine natürliche Zahl). Mit einem - insbesondere jedem - Teilbereich kann mindestens ein Programmparameter oder ein bestimmter Wert eines Programmparameters assoziiert sein. Die Steuereinrichtung ist dazu ausgebildet, eine Benutzerbewegung zu erfassen, deren Darstellung als grafisches Skizzenelement auf dem Anzeigenbereich mindestens einen oder mindestens zwei Teilbereiche berührt, und dadurch mindestens einen Programmparameter auswählt, der mit dem Teilbereich assoziiert ist. Durch die Benutzerbewegung können so mehrere Arbeitsstationen ausgewählt werden, insbesondere in einer Reihenfolge verknüpft werden, die z.B. der Reihenfolge entsprechen kann, in der eine kontinuierliche Benutzerbewegung diese Teilbereiche berührt. Auf diese Weise können Arbeitsstationen, insbesondere die Reihenfolge ihrer Verwendung in einem Behandlungsprozess festgelegt werden. Für den Benutzer ergibt sich der Vorteil einer effizienten Prozessplanung.

Bei einem Pipettierautomaten ergibt sich eine weitere bevorzugte Ausführungsform. Die Eingabe mittels Benutzerbewegung kann dazu genutzt werden, um bei einem als Pipettierautomat ausgeführten erfindungsgemäßen Laborgerät ein Pipettiermuster zu definieren. Ein Pipettierautomat weist mindestens einen Arbeitsbereich mit mindestens einer oder mehrerer Arbeitsstationen auf. Die Arbeitsstationen werden entweder automatisch oder manuell mit Laborgefäßen bestückt, z.B. mit Einzelgefäßen, z.B. PCR-Tubes, oder mit Mehrfach-Laborgefäßen, z.B. Mikrotiterplatten. Die Laborgefäße werden ebenfalls automatisch oder manuell zumindest teilweise mit flüssigen Laborproben befüllt. Die Aufgabe des Pipettierautomaten kann es dann sein, nach einem bestimmten Pipettiermuster die flüssigen Laborproben aus einer ersten Menge an Laborgefäßen gemäß einem vom Anwender zu wählenden Pipettiermuster in eine zweite Menge an Laborgefäßen zu pipettieren. Die erste und zweite Menge an Laborgefäßen kann jeweils mindestens ein oder mehrere Laborgefäße aufweisen.

Die Steuereinrichtung ist vorzugsweise dazu eingerichtet, im Anzeigebereich ein erste Menge an grafischen Symbolen darzustellen, welche eine erste Menge an Laborgefäßen repräsentieren, und eine zweite Menge an grafischen Symbolen darzustellen, welche eine zweite Menge an Laborgefäßen repräsentieren, wobei die erste Menge und zweite Menge an Symbolen insbesondere voneinander beabstandet angeordnet sind. Die Steuereinrichtung ist vorzugsweise dazu eingerichtet, eine Benutzerbewegung zu erfassen, deren Darstellung als grafisches Skizzenelement die erste Menge an grafischen Symbolen berührt und zudem eine zweite Menge an grafischen Symbolen berührt, so dass die erste und zweite Menge an grafischen Symbolen durch das grafische Skizzenelement grafisch miteinander verbunden sind. Die Steuereinrichtung ist vorzugsweise dazu eingerichtet, auf diese Weise Programmparameter und/oder deren Werte festzulegen, welche beim automatisierten Pipettieren festlegen, gemäß welchem Pipettiermuster die flüssigen Proben aus der ersten Menge an Laborgefäßen - nämlich Ansaugen der ersten Laborproben in mindestens ein Flüssigkeitstransferwerkzeug, z.B. mindestens eine Pipettierspitze - in die zweite Menge an Laborgefäßen -nämlich Abgeben der Laborproben aus dem mindestens einen Flüssigkeitstransferwerkzeug in die zweite Menge an Laborgefäßen- pipettiert werden. Das Pipettiermuster kann mehrere Flüssigkeitstransfers von Laborproben aus einer ersten Menge an Laborgefäßen in eine zweite Menge an Laborgefäßen vorsehen. Die Steuereinrichtung ist vorzugsweise dazu eingerichtet, mehrere Benutzerbewegungen sukzessive zu erfassen, welche jeweils mindestens einen Pipettiervorgang repräsentieren können.

Vorzugsweise verfügt die Steuereinrichtung oder die Benutzerschnittstellensteuerung über eine Gestendatenbank, in der Gesteninformationen zur Erkennung vorbestimmter Gesten gespeichert sind, insbesondere bezeichnet als Gestendaten. Die Gestendatenbank kann in einer Speichereinrichtung der Steuereinrichtung gespeichert sein. Vorzugsweise ist die Steuereinrichtung oder die Benutzerschnittstellensteuerung dazu eingerichtet, aus der Benutzerbewegung die Bewegungsinformationen über diese Benutzerbewegung zu ermitteln, insbesondere in Form von Bewegungsdaten.

Vorzugsweise verfügt die Steuereinrichtung oder die Benutzerschnittstellensteuerung über Korrelationsdaten zwischen Gestendaten und anderen Parametern, insbesondere Programmparametern. Diese Korrelationsdaten können in der Speichereinrichtung gespeichert sein, insbesondere in einer Datenbank, insbesondere in der Gestendatenbank. Diese Korrelation erfolgt vorzugsweise kontextabhängig, d.h. In Abhängigkeit vom Status des Steuerprogramms des Laborgeräts wird vorzugsweise eine damit verknüpfte Korrelation zwischen Geste und Parameter vorgenommen. Der Status kann z.B. durch die dem Benutzer im Anzeigebereich angezeigte Eingabemaske oder einen bestimmten Eingabemodus, während dem die Erfassung von Benutzerbewegungen ausschließlich ermöglicht sein kann, bestimmt sein. Diese Eingabemaske kann je nach Programmausführung des Steuerprogramms bzw. des Programmmoduls oder Methodenprogramms bzw. je nach Status von dessen Abarbeitung unterschiedlich sein. Infolgedessen kann eine vertikale Geste während eines ersten Status des Steuerprogramms die Auswahl eines Zeitparameters und Festlegen von dessen Wert bewirken, während dieselbe vertikale Geste während eines zweiten Status des Steuerprogramms die Auswahl eines Ortsparameters und Festlegen von dessen Wert bewirken kann.

Die Bewegungsdaten können in einer Speichereinrichtung der Steuereinrichtung kurzzeitig oder langfristig speicherbar sein, insbesondere kann die Steuereinrichtung dazu ausgebildet sein, einem zuvor - insbesondere mittels eines Authentifizierungsvorgang am Laborgerät - identifizierten Benutzer bestimmte Bewegungsdaten zuzuordnen und diese mit der Identität des Benutzers als Benutzerdatensatz zu speichern.

Vorzugsweise ist die Steuereinrichtung dazu ausgebildet, ein Gestentrainingsverfahren auszuführen, das die Durchführung vorbestimmter Gesten von einem individuellen Benutzer oder aber von einem Administrator des Laborgeräts am Laborgerät erfordert. Dazu wird vorzugsweise dem Benutzer mindestens ein nachzubildendes Bewegungsmuster im Display angezeigt; dieses Bewegungsmuster wird dann vom Benutzer nachgebildet, wobei die Benutzerbewegungen erfasst und als benutzerabhängige Bewegungsdaten gespeichert werden. Mittels eines solchen Gestentrainingsverfahrens lässt sich die Gestenerkennungswahrscheinlichkeit optimieren, mit der eine Benutzerbewegung korrekt als eine bestimmte Geste identifiziert wird. Der Grund liegt darin, dass die Gestik der Menschen, ähnlich der Sprache oder Handschrift, eine individuelle Eigenschaft ist, deren individuelle Auswertung einer benutzer-unspezifischen Auswertung überlegen sein kann.

Alternativ oder zusätzlich zu dem zuvor beschriebenen Gestentrainingsverfahren kann ein weiteres Gestentrainingsverfahren eingesetzt werden, mit dem der Benutzer -und zwar vorzugsweise jeder Benutzer- mindestens einmal oder mehrmals zur Durchführung einer vorbestimmten Geste trainiert wird, bis er zur Gesteneingabe am Laborgerät zertifiziert ist. Dazu kann eine Geste einmal oder wiederholt vorgegeben und vom Benutzer nachgebildet werden, bis die Erkennung ausreichend sicher ist.

Die Bewegungsinformationen sind insbesondere geeignet, um mit den Gesteninformationen verglichen zu werden. Vorzugsweise ist die Steuereinrichtung oder die Benutzerschnittstellensteuerung dazu eingerichtet, die Bewegungsinformationen mit Gesteninformationen zu vergleichen. Das Ergebnis dieses Vergleichs kann festlegen, ob eine vorbestimmte Geste im Wesentlichen eindeutig erkannt wurde, oder mit einer bestimmten Wahrscheinlichkeit erkannt wurde. Das Ergebnis dieses Vergleichs kann festlegen, welche vorbestimmte Geste vom Benutzer durchgeführt wurde.

Eine Steuereinrichtung weist im Rahmen der vorliegenden Erfindung generell insbesondere eine Datenverarbeitungseinrichtung, insbesondere eine Recheneinheit (CPU) zum Verarbeiten von Daten und/oder einen Mikroprozessor auf oder ist eine Datenverarbeitungseinrichtung. Eine Recheneinheit der Steuereinrichtung eines Laborgeräts ist vorzugsweise auch zum Steuern des Behandlungsprozesses und/oder der individuellen Behandlungen eingerichtet.

Der Begriff "Behandlung" bedeutet insbesondere, dass eine Laborprobe, die zumeist flüssig ist, bewegt, und/oder transportiert, und/oder untersucht und/oder verändert wird, insbesondere in ihrer Zusammensetzung, physikalisch, chemisch, biochemisch oder auf andere Weise verändert wird.

Die Steuereinrichtung des Laborgeräts und/oder die Benutzerschnittstelleneinrichtung können -insbesondere alle- in einer physikalischen Geräteinheit integriert sein, können aber jeweils auch eine eigene physikalischen Geräteinheit sein. Eine physikalische Geräteinheit kann insbesondere ein Modul sein, das mit dem Laborgerät verbunden ist oder verbindbar ist. Die Steuereinrichtung des Laborgeräts und/oder die Benutzerschnittstelleneinrichtung oder Bestandteile dieser Komponenten können auch durch Softwarefunktionen implementiert sein, oder können insbesondere als Programmcode vorliegen. Ein Laborgerät kann z.B. einen Computer aufweisen, der in Kombination mit Softwarefunktionen die Steuereinrichtung des Laborgeräts und/oder die Benutzerschnittstelleneinrichtung jeweils zumindest teilweise implementiert. Ist z.B. die Benutzerschnittstelleneinrichtung in das Laborgerät integriert, kann die Benutzerschnittstelleneinrichtung teilweise selber Teil der Steuereinrichtung des Laborgeräts sein bzw. teilweise mittels der Steuereinrichtung implementiert sein, insbesondere durch Softwarefunktionen, insbesondere zumindest teilweise als ausführbarer Programmcode.

Ein Modul kann insbesondere die Benutzerschnittstelleneinrichtung aufweisen. Ein Modul ist ein von anderen Geräten getrenntes, und/oder ein vom anderen Gerät, insbesondere Laborgerät, abtrennbares Gerät. Ein Laborgerät kann eine Verbindungseinrichtung aufweisen, durch die das Modul mit dem Laborgerät verbindbar ist, insbesondere mittels einer vom Benutzer lösbaren Verbindung. Ein Modul kann portabel sein, also von einem Benutzer transportierbar. Das Modul kann auch fest mit dem Laborgerät verbunden sein. Die modulare Bauweise bietet Vorteile bei der Herstellung von Laborgeräten. Ein portables Modul bietet eine größere Flexibilität bei der Benutzung eines Laborgeräts.

Die Datenverarbeitungseinrichtung weist vorzugsweise eine Recheneinheit auf, insbesondere eine CPU, ferner vorzugsweise mindestens eine Datenspeichereinrichtung, insbesondere zur flüchtigen und/oder dauerhaften Speicherung von Daten. Die Datenverarbeitungseinrichtung ist vorzugsweise dazu ausgebildet, über die erste Schnittstelleneinrichtung eine oder mehrere erste Datenverbindungen mit einer oder mehreren Benutzerschnittstelleneinrichtungen herzustellen, die insbesondere Bestandteile des Laborgerätes sein können; vorzugsweise über die zweite Schnittstelleneinrichtung eine zweite Datenverbindung mit dem Laborgerät herzustellen.

Eine Schnittstelleneinrichtung dient der Verbindung von zwei Einrichtungen, die jeweils Signale, insbesondere Informationen, insbesondere Daten, verarbeiten können, insbesondere senden und/oder empfangen können. Eine Schnittstelleneinrichtung kann mindestens eine Hardwareschnittstelle beinhalten und/oder mindestens eine Softwareschnittstelle.

Hardwareschnittstellen sind insbesondere Schnittstellen zwischen elektrisch arbeitenden Einheiten, gemäß dem üblichen Verständnis in der Elektrotechnik und Elektronik. Vorliegend bezeichnet der Begriff "Hardwareschnittstelle" insbesondere auch die Verbindungskornponenten zwischen wenigstens zwei elektrisch arbeitenden Einheiten selbst, also insbesondere alle Bestandteile, die diese Verbindung ermöglichen, z.B. integrierte Schaltkreise, Elektronik und Leitungen, über die zwischen den wenigstens zwei elektrisch arbeitenden Einheiten elektrische Signale versandt werden. Diese zwei elektrisch arbeitenden Einheiten können insbesondere ein Laborgerät und eine externe Datenverarbeitungseinrichtung sein, oder zwei Laborgeräte, oder zwei elektrisch arbeitenden Einheiten innerhalb eines Laborgeräts. Eine Hardwareschnittstelle muss nicht, aber kann eine lösbare Verbindungseinrichtung zum Lösen und/oder Wiederherstellen dieser Verbindung aufweisen, insbesondere mindestens einen Stecker.

Softwareschnittstellen, insbesondere softwareseitige Datenschnittstellen, sind insbesondere logische Berührungspunkte in einem Informationsverwaltungssystem, insbesondere Softwaresystem: Sie ermöglichen und regeln den Austausch von Kommandos und Daten zwischen verschiedenen Prozessen und Komponenten.

Softwareschnittstellen können nur zur Kommunikation benutzte, datenorientierte Schnittstellen sein. In diesem Fall enthält die Softwareschnittstelle lediglich die Informationen, die zwischen beteiligten Systemteilen ausgetauscht werden.

Der Begriff "gerätegesteuerte Behandlung" bedeutet, dass die Behandlung der mindestens einen Laborprobe zumindest teilweise vom Laborgerät gesteuert wird, insbesondere durchgeführt wird. Soweit die Behandlung vom Laborgerät gesteuert und/oder durchgeführt wird, wird diese insbesondere insofern nicht vom Benutzer gesteuert und/oder durchgeführt, insbesondere nicht manuell vom Benutzer gesteuerte und/oder durchgeführt.

Unter einer gerätegesteuerten Behandlung wird ferner vorzugsweise verstanden, dass die Behandlung in Abhängigkeit von mindestens einer Benutzereingabe zumindest teilweise vom Laborgerät gesteuert wird, insbesondere durchgeführt wird. Die Benutzereingabe kann vor dem Start der Behandlung erfolgen und/oder während der Behandlung. Die Benutzereingabe erfolgt vorzugsweise über eine Benutzerschnittstelleneinrichtung, die vorzugsweise ein Bestandteil des Laborgeräts ist, oder die separat vom Laborgerät vorgesehen ist und mit der Steuereinrichtung des Laborgeräts signalverbunden ist. Die Benutzereingabe dient insbesondere der Eingabe mindestens eines Parameters, dessen Wert die Behandlung beeinflusst und/oder steuert. Dieser Parameter kann insbesondere ein Programmparameter sein.

Die "gerätegesteuerte Behandlung" bezeichnet insbesondere die zumindest teilautomatisierte Behandlung. Bei einer teilautomatisierten Behandlung ist es insbesondere möglich, dass die Behandlung so durchgeführt wird, dass nach dem Starten der Behandlung und vor dem Beenden der Behandlung mindestens eine Benutzereingabe erfolgt, mit der der Benutzer die laufende Behandlung beeinflussen kann, insbesondere indem er z.B. eine über eine Benutzerschnittstelleneinrichtung des Laborgeräts erfolgende automatische Abfrage beantwortet, insbesondere eine Eingabe bestätigt oder verneint oder andere Eingaben vornimmt. Bei der teilautomatisierten Behandlung ist es insbesondere möglich, dass die Behandlung mehrere Behandlungsschritte aufweist, die insbesondere zeitlich nacheinander automatisch durchgeführt werden, und mindestens einen Behandlungsschritt aufweist, der eine, insbesondere über eine Benutzerschnittstelleneinrichtung erfolgende, Benutzereingabe erfordert.

Eine gerätegesteuerte Behandlung ist vorzugsweise eine programmgesteuerte Behandlung, also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mithilfe einer digitalen Datenverarbeitungseinrichtung, die insbesondere Bestandteil der Steuereinrichtung des Laborgeräts sein kann. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Ein Programmparameter kann ein benutzerseitig erforderlicher Programmparameter sein. Ein benutzerseitig erforderlicher Programmparameter zeichnet sich dadurch aus, dass er für die Ausführung einer Behandlung, insbesondere zur Ausführung eines Methodenprogramms, erforderlich ist. Andere Programmparameter, die nicht benutzerseitig erforderlich sind, können aus den benutzerseitig erforderlichen Programmparametern abgeleitet werden oder anderweitig verfügbar gemacht werden, insbesondere wahlweise vom Benutzer eingestellt werden. Die Einstellung eines Programmparameters durch einen Benutzer erfolgt insbesondere durch Anzeige einer Auswahl von möglichen vorgegebenen Werten aus einer im Laborgerät gespeicherten Liste von vorgegebenen Werten, wobei der Benutzer aus dieser Liste den gewünschten Parameter auswählt und somit einstellt. Es ist auch möglich, dass dieser Programmparameter eingestellt wird, indem der Benutzer den Wert eingibt, indem er z.B. über einen Ziffernblock eine Zahl eingibt, die dem gewünschten Wert entspricht oder indem der Benutzer einen Wert kontinuierlich oder in Inkrementen erhöht bzw. erniedrigt, bis dieser dem gewünschten Wert entspricht, und den Wert so einstellt. Andere Formen der Eingabe, z.B. durch Sprachsteuerung und/oder Gestensteuerung sind denkbar.

Unter einem Programm wird insbesondere ein Computerprogramm verstanden. Ein Programm ist eine Folge von Anweisungen, insbesondere bestehend aus Deklarationen und Instruktionen, um auf einer digitalen Datenverarbeitungsanlage eine bestimmte Funktionalität, Aufgaben- oder Problemstellung bearbeiten und/oder lösen zu können. Ein Programm liegt in der Regel als Software vor, die mit einer digitalen Datenverarbeitungsanlage verwendet wird. Das Programm kann insbesondere als Firmware vorliegen, im Fall der vorliegenden Erfindung insbesondere als Firmware der Steuereinrichtung des Laborgeräts. Das Programm liegt meist auf einem Datenträger als ausführbare Programmdatei, häufig im sogenannten Maschinencode vor, die zur Ausführung in den Arbeitsspeicher des Rechners der digitalen Datenverarbeitungsanlage geladen wird. Das Programm wird als Abfolge von Maschinen-, d. h. Prozessorbefehlen von dem/den Prozessoren des Computers verarbeitet und damit ausgeführt. Unter ,Computerprogramm wird insbesondere auch der Quelltext des Programms verstanden, aus dem im Verlauf der Steuerung des Laborgerätes der ausführbare Code entstehen kann.

Als Anweisung wird in üblicher Weise ein zentrales Element einer Programmiersprache bezeichnet. Die Programme derartiger Sprachen setzen sich primär aus einer oder mehreren Anweisungen zusammen. Eine Anweisung stellt eine in der Syntax einer Programmiersprache formulierte einzelne Vorschrift dar, die im Rahmen der Abarbeitung des Programms auszuführen ist. Wie eine Anweisung syntaktisch auszusehen hat, wird durch die jeweilige Programmiersprache bzw. deren Spezifikation festgelegt. In der maschinennahen Programmierung werden Anweisungen häufig auch als Befehl bezeichnet. Anweisungen sind üblicherweise Zuweisungen, Kontrollanweisungen (wie Sprünge, Schleifen und bedingte Anweisungen) und Prozeduraufrufe. Abhängig von der Programmiersprache sind teilweise auch Zusicherungen, Deklarationen, Klassen- und Funktionsdefinitionen Anweisungen. Die Anweisungen des Steuerprogramms können so in üblicher Weise ausgestaltet sein.

Unter einem Programmmodul wird in üblicher Weise eine abgeschlossene funktionale Einheit einer Software verstanden, bestehend aus einer Folge von Verarbeitungsschritten und Datenstrukturen. Dabei können insbesondere folgende Definitionen zutreffen: Der Inhalt eines Moduls ist häufig eine wiederkehrende Berechnung oder Bearbeitung von Daten, die mehrfach durchgeführt werden muss. Module bieten eine Kapselung durch die Trennung von Schnittstelle und Implementierung: Die Schnittstelle eines Moduls definiert die Datenelemente, die als Eingabe und Ergebnis der Verarbeitung durch das Modul benötigt werden. Die Implementierung enthält den tatsächlichen Programmcode. Ein Modul wird zum Beispiel als Funktion oder Unterprogramm aufgerufen, führt eine Reihe von Verarbeitungsschritten durch und liefert als Ergebnis Daten zurück an das aufrufende Programm. Ein Modul kann selbst weitere Module aufrufen - so ist eine Hierarchie von Programmaufrufen möglich. Die in Modulen festgelegten Datenstrukturen und Methoden können gegebenenfalls vererbt und von anderen Modulen geerbt werden. Module sind daher ein wesentliches Element in der strukturierten und objektorientierten Programmierung.

Unter einem Steuerprogramm wird ein ausführbares Computerprogramm verstanden, das vorzugsweise die gewünschte Behandlung der mindestens einen Probe steuert und/oder durchführt, insbesondere in Abhängigkeit von mindestens einem Programmparameter. Dieser Programmparameter kann ein vom Benutzer beeinflusster und/oder eingestellter Programmparameter sein. Die Behandlung kann insbesondere gesteuert werden, indem die Steuereinrichtung in Abhängigkeit von den Programmparametern einen oder mehrere Steuerparameter erzeugt, mittels derer die mindestens eine Behandlungseinrichtung gesteuert wird. Vorzugsweise weist das Laborgerät ein Betriebssystem auf, das ein Steuerprogramm sein kann oder aufweisen kann. Das Steuerprogramm kann insbesondere ein Betriebssystem des Laborgeräts bezeichnen oder einen Bestandteil des Betriebssystems. Das Betriebssystem steuert die Behandlung und weitere Betriebsfunktionen des Laborgeräts.

Das Steuerprogramm kann insbesondere mit der Benutzerschnittstelleneinrichtung signalverbunden sein, und/oder kann die Benutzerschnittstelleneinrichtung steuern. Die Steuereinrichtung der Benutzerschnittstelleneinrichtung kann in die Steuereinrichtung des Laborgeräts integriert sein, oder kann getrennt von dieser Steuereinrichtung ausgebildet sein. Die Steuereinrichtung der Benutzerschnittstelleneinrichtung kann in die Steuerung des Laborgeräts integriert sein, kann vom Steuerprogramm steuerbar sein und/oder kann insbesondere in das Steuerungsprogramm integriert sein. Das Steuerprogramm kann weitere vorzugsweise vorgesehene Funktionen des Laborgeräts steuern, zum Beispiel eine Energiesparfunktion des Laborgeräts oder eine Kommunikationsfunktion zur Kommunikation mit externen Datenverarbeitungseinrichtungen, die insbesondere separat zum Laborgerät vorgesehen sind und insbesondere nicht ein Bestandteil des Laborgeräts sind.

Unter einem Methodenprogramm wird ein Programm verstanden, das den konkreten Ablauf einer Behandlung bestimmt, insbesondere gemäß einer vorbestimmten Behandlungsart und/oder gemäß einer benutzerseitig festgelegten Weise.

Der Begriff Laborgerät bezeichnet insbesondere ein Gerät, das zur gerätegesteuerten Behandlung mindestens einer Laborprobe ausgebildet ist und das zur Verwendung in einem Labor ausgebildet ist. Bei diesem Labor kann es sich insbesondere um ein chemisches, biologisches, biochemisches, medizinisches oder forensisches Laboratorium handeln. Solche Labors dienen der Forschung und/oder der Analyse von Laborproben, können aber auch zur Herstellung von Produkten mittels Laborproben oder der Herstellung von Laborproben dienen.

Ein Laborgerät ist vorzugsweise eines der folgenden Laborgeräte und/oder ist vorzugsweise als mindestens eines der folgenden Laborgeräte ausgebildet: Laborzentrifuge, im Rahmen der Beschreibung der vorliegenden Erfindung auch bezeichnet als "Zentrifuge" oder "centrifuge"; Thermocycler, im Rahmen der Beschreibung der vorliegenden Erfindung auch bezeichnet als "Cycler"; Labor-Spektralphotometer, im Rahmen der Beschreibung der vorliegenden Erfindung auch bezeichnet als "Biospektrometer"; Zellenzählgerät, im Rahmen der Beschreibung der vorliegenden Erfindung auch bezeichnet als "cellcounter", insbesondere optische Zählgeräte; Labor-Inkubator, im Rahmen der Beschreibung der vorliegenden Erfindung auch bezeichnet als "incubator"; Labor-Schüttelgerät, im Rahmen der Beschreibung der vorliegenden Erfindung auch bezeichnet als "shaker"; Labor-Mischer, auch bezeichnet als "mixing device"; Labor-Gefriergerät, im Rahmen der Beschreibung der vorliegenden Erfindung auch bezeichnet als "freezer"; Bioreaktor, im Rahmen der Beschreibung der vorliegenden Erfindung auch bezeichnet als Fermenter; Sicherheitswerkbank, insbesondere Biosicherheitswerkbank, im Rahmen der Beschreibung der vorliegenden Erfindung auch bezeichnet als "biosafety cabinet"; Probenplatten-Lesegerät, im Rahmen der Beschreibung der vorliegenden Erfindung auch bezeichnet als "plate reader", insbesondere "microplate reader"; Laborautomat zur Behandlung von fluiden Proben, insbesondere Pipettierautomat;.

Eine Laborzentrifuge ist ein Gerät, das unter Ausnutzung der Massenträgheit arbeitet. Die Laborzentrifuge, insbesondere die Behandlungseinrichtung der Laborzentrifuge, weist insbesondere mindestens einen Rotor auf, in dem die mindestens eine Laborprobe anordenbar ist. Der mindestens eine Rotor ist rotierbar in mindestens einem Zentrifugenkessel angeordnet. Die Laborzentrifuge, insbesondere die Behandlungseinrichtung der Laborzentrifuge, weist mindestens eine Antriebseinrichtung auf, mittels der die Rotation angetrieben und/oder gebremst wird. Die Proben sind in dem mindestens einen Rotor anordenbar, vorzugsweise in Laborbehältern, z.B. Probenröhrchen, die in geeigneten Halterungen im Rotor angeordnet werden. Vorzugsweise weist die Laborzentrifuge, insbesondere die Behandlungseinrichtung der Laborzentrifuge, mindestens eine Heiz-/Kühleinrichtung auf, mit der die Temperatur der mindestens einen im Rotor angeordneten Probe gesteuert und/oder geregelt werden kann. Vorzugsweise weist die Laborzentrifuge, insbesondere die Behandlungseinrichtung der Laborzentrifuge, eine Zeitgebereinrichtung auf, mit der zeitliche Parameter der Rotation oder Temperatureinstellung steuerbar sind. Die Funktionsweise beruht auf der Zentrifugalkraft, die aufgrund einer gleichförmigen Kreisbewegung der zu zentrifugierenden Proben zustande kommt. Die Zentrifugalkraft wird zur Stofftrennung von Stoffen unterschiedlicher Dichte genutzt, die in einer Probe enthalten sind. Eine Zentrifuge kann ein Trennverfahren durchführen, bei dem insbesondere die Bestandteile von Suspensionen, Emulsionen und/oder Gasgemischen getrennt werden. Die gerätegesteuerte Behandlung der mindestens einen Laborprobe entspricht bei einer Laborzentrifuge einer Rotationsbehandlung, der die mindestens eine Probe unterzogen wird. Mögliche Parameter, insbesondere Programmparameter, insbesondere Nutzerparameter, die zur Beeinflussung einer Rotationsbehandlung verwendet werden, definieren insbesondere eine Temperatur der Laborzentrifuge, eine Rotationsgeschwindigkeit der Laborzentrifuge, einen zeitlichen Parameter der Rotation oder Temperatureinstellung, und/oder mindestens einen Ablaufparameter, der den Ablauf, insbesondere die Reihenfolge, eines aus mehreren Rotationsschritten bestehenden Rotationsprogramms beeinflusst oder definiert. Die Temperatur der Laborzentrifuge kann insbesondere mindestens eine Temperatur im Inneren des mindestens einen Rotors sein, insbesondere mindestens eine Temperatur mindestens einer Probe.

Ein Thermocycler ist ein Gerät, das in der Lage ist, die Temperatur mindestens einer Probe zeitlich nacheinander auf eine vorbestimmte Temperatur einzustellen und für eine vorgegebene Dauer auf dieser Temperaturstufe zu halten. Der Ablauf dieser Temperatursteuerung ist zyklisch. Das heißt ein vorbestimmter Temperaturzyklus, also eine Abfolge von mindestens zwei Temperaturstufen, wird wiederholt durchgeführt. Dieses Verfahren dient insbesondere der Durchführung einer Polymerase-Kettenreaktion (PCR). In diesem Zusammenhang bezeichnet man einen Thermocycler auch manchmal als PCR-Block. Ein Thermocycler, insbesondere die Behandlungseinrichtung des Thermocyclers, weist vorzugsweise einen Thermoblock auf. Ein Thermoblock ist ein Probenhalter aus einem wärmeleitenden Material, meistens ein metallhaltiges Material oder ein Metall, insbesondere Aluminium oder Silber. Der Probenhalter weist eine Kontaktierseite auf, die durch mindestens eine Heiz-/Kühleinrichtung des Thermocyclers, insbesondere ein Peltierelement, kontaktiert wird. Der Thermocycler, insbesondere die Behandlungseinrichtung des Thermocyclers, weist eine Regeleinrichtung mit mindestens einem Regelkreis auf, dem als Stellglied die mindestens eine Heiz-/Kühleinrichtung und als Messglied mindestens eine Temperaturmesseinrichtung zugeordnet sind. Mittels der Regeleinrichtung wird die Temperatur einer Temperaturstufe geregelt. Ein Kühlkörper des Thermocyclers, insbesondere der Behandlungseinrichtung des Thermocyclers, dient zur Kühlung von Abschnitten des Thermocyclers, insbesondere der Kühlung der Peltierelemente. Der Thermocycler, insbesondere die Behandlungseinrichtung des Thermocyclers, kann weitere Heiz- und/oder Kühlelemente aufweisen. Vorzugsweise weist der Thermocycler, insbesondere die Behandlungseinrichtung des Thermocyclers, eine Zeitgebereinrichtung auf, mit der zeitliche Parameter der Einstellung des Temperaturzyklus steuerbar sind. Die gerätegesteuerte Behandlung der mindestens einen Laborprobe entspricht bei einem Thermocycler einer Temperaturzyklusbehandlung, der die mindestens eine Probe unterzogen wird. Mögliche Parameter, insbesondere Programmparameter, insbesondere Nutzerparameter, die zur Beeinflussung einer Temperaturzyklusbehandlung verwendet werden, definieren insbesondere die Temperatur einer Temperaturstufe, die Dauer einer Temperaturstufe, die Steuerung weiterer Heiz- und/oder Kühlelemente, und/oder die Anzahl von Temperaturstufen oder Zyklen, und/oder mindestens einen Ablaufparameter, der den Ablauf, insbesondere die Reihenfolge, eines aus mehreren Schritten bestehenden Temperaturkontrollprogramms beeinflusst oder definiert.

Ein Labor-Spektralphotometer ist ein Gerät, das durch das Beleuchten mindestens eines Messvolumens mindestens einer Laborprobe meist über das gesamte Spektrum von infrarot bis ultraviolett des sichtbaren Lichtes die Remissionswerte ermittelt. Die Remission bezeichnet die Situation, dass ein Messvolumen einen Teil des Lichtspektrums absorbiert und einen Teil des Spektrums transmittiert (transparente Medien) bzw. reflektiert (undurchsichtige Medien). Mit dem Labor-Spektralphotometer wird insbesondere das Absorptionsvermögen einer Probe in Abhängigkeit von der Lichtwellenlänge gemessen. Darüber hinaus besteht insbesondere die Möglichkeit, den Anwendungsbereich des Labor-Spektralphotometers durch verschiedene Module zu erweitern. So ist z.B. die Anordnung eines Fluoreszenz-Moduls zur Messung von Fluoreszenz oder eines Temperiermoduls zur Temperierung der Probe im Spektrometer denkbar. Das gemessene Absorptionsspektrum enthält insbesondere die bei bestimmten Wellenlängen gemessenen Lichtintensitäten. Das Absorptionsspektrum ist charakteristisch für die Laborprobe bzw. den darin enthaltenen Stoff oder die Stoffe. Dies kann zur qualitativen Analyse der Laborprobe genutzt werden. Ist die flüssige Probe bzw. der darin gelöste Stoff bekannt, kann durch Messung der Absorption die Konzentration des gelösten Stoffes ermittelt werden. Dies kann zur quantitativen Analyse der Laborprobe genutzt werden. Das Labor-Spektralphotometer, insbesondere die Behandlungseinrichtung des Labor-Spektralphotometers, weist vorzugsweise mindestens eine Lichtquelle auf, vorzugsweise mindestens einen Zeitgeber, vorzugsweise mindestens einen Photodetektor. Die gerätegesteuerte Behandlung der mindestens einen Laborprobe entspricht bei einem Labor-Spektralphotometer einer Licht- und Messbehandlung, der die mindestens eine Probe unterzogen wird. Mögliche Parameter, insbesondere Programmparameter, insbesondere Nutzerparameter, die zur Beeinflussung einer Licht- und Messbehandlung verwendet werden, definieren insbesondere das optische Lichtspektrum, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms beeinflusst oder definiert.

Ein Zellenzählgerät dient dem Zählen von biologischen Zellen, bzw. Partikeln, die in einer Laborprobe enthalten sind. Es gibt verschiedene physikalische Prinzipien, die zum Zählen von Zellen in Frage kommen, insbesondere optische Verfahren, bei denen die zu messende Laborprobe in einer Zählkammer angeordnet wird, insbesondere bei automatisch arbeitenden zusätzlich beleuchtet wird und ein Bild der in der Zählkammer angeordneten Zellen bzw. Partikel erfasst wird und ausgewertet wird. Ein weiteres etabliertes Verfahren ist die Impedanzmessung: ein als Coulter Counter ausgeführtes Zellenzählgerät leitet die die Zellen enthaltende Laborprobe durch eine Apertur ("Messschleuse"). Jeder Durchtritt einer Zelle durch die Apertur wird als zählbares Ereignis elektrisch detektiert. Optische Zellenzählgeräte, insbesondere die Behandlungseinrichtung des Zellenzählgeräts, weisen je nach Ausführung vorzugsweise mindestens eine Lichtquelle auf, mindestens eine Bilderfassungseinrichtung und mindestens eine Bildauswerteeinrichtung*, zusätzlich u.U. eine Positioniereinrichtung. Die gerätegesteuerte Behandlung der mindestens einen Laborprobe entspricht bei einem optischen Zellenzählgerät z.B. einer Licht- und Messbehandlung, bei einem nach Coulter Prinzip arbeitenden Gerät einer Pump- und Messbehandlung, der die mindestens eine Probe unterzogen wird. Mögliche Parameter, insbesondere Programmparameter, insbesondere Nutzerparameter, die zur Beeinflussung einer Licht- und Messbehandlung oder der Pump- und Messbehandlung verwendet werden, definieren insbesondere die Lichtintensität der Lichtquelle, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms oder Pump- und Messbehandlungsprogramms beeinflusst oder definiert. Bei optischen Zählgeräten sind außerdem die für die Bildauswertung notwendigen Algorithmen, deren Reihenfolge und Parametrierung entscheidend für die Aussagekraft des Messergebnisses. Optische Messgeräte, aber auch Coulter Counter, nutzen oft Zählkammern zum einmaligen Gebrauch ("Consumables)", dies sind den herkömmlichen Neubauerzählkammern nachempfundene Kunststoffartikel, bzw., bei den Coulter Countern, "Lab-on-a-Chip"- ähnliche Einmalzählkammern. Es gibt aber auch Geräte, die ohne diese Verbrauchsmaterialien arbeiten (z.B. "CASY").

Ein Labor-Inkubator ist ein Gerät, mit dem kontrollierte Klimabedingungen für verschiedene biologische Entwicklungs- und Wachstumsprozesse geschaffen und erhalten werden können. Er dient der Schaffung und Erhaltung eines Mikroklimas mit geregelten Gas-, und/oder Luftfeuchtigkeits- und/oder Temperatur-Bedingungen in einem Inkubatorraum, wobei diese Behandlung zeitabhängig sein kann. Der Labor-Inkubator, insbesondere die Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines dem Inkubatorraum zugeführten Austauschgases, insbesondere Frischluft, eine Einstelleinrichtung für die Zusammensetzung des Gases im Inkubatorraum des Labor-Inkubators, insbesondere zur Einstellung des CO₂ und/oder des O₂ Gehalts des Gases und/oder eine Einstelleinrichtung zur Einstellung der Luftfeuchtigkeit im Inkubatorraum des Labor-Inkubators. Der Labor-Inkubator, insbesondere die Behandlungseinrichtung des Labor-Inkubators, weist insbesondere den Inkubatorraum auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. CO₂-Inkubatoren dienen insbesondere der Kultivierung tierischer bzw. humaner Zellen. Inkubatoren können Wendevorrichtungen zum Wenden der mindestens einen Laborprobe und/oder eine Schütteleinrichtung zum Schütteln bzw. Bewegen der der mindestens einen Laborprobe aufweisen. Die gerätegesteuerte Behandlung der mindestens einen Laborprobe entspricht bei einem Labor-Inkubator einer Klimabehandlung, der die mindestens eine Probe unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, den O₂- und/oder CO₂-Partialdruck im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Ein Labor-Schüttelgerät dient der Bewegung einer Laborprobe, insbesondere zum Mischen einer mehrere Bestandteile aufweisenden Laborprobe. Labor-Schüttelgeräte gibt es in verschiedenen Ausführungen, insbesondere als Überkopf-Schüttelgeräte oder als Flachbett-Schüttelgeräte. Labor-Schüttelgeräte können eine Temperierfunktion zum Temperieren der mindestens einen Laborprobe aufweisen, und können insbesondere eine Inkubatorfunktion zum Inkubieren der mindestens einen Laborprobe bei kontrollierten Klimabedingungen aufweisen. Labor-Schüttelgeräte, insbesondere deren Behandlungseinrichtung, können insbesondere zur Durchführung einer oszillierenden Bewegung eingerichtet sein. Labor-Schüttelgeräte, insbesondere deren Behandlungseinrichtung, weisen insbesondere einen Antrieb zum Antreiben der Bewegung auf, weisen insbesondere eine Zeitgebereinrichtung auf, mit der zeitliche Parameter der Einstellung der Schüttelbehandlung steuerbar sind und weisen insbesondere mindestens eine Heiz-/Kühleinrichtung und mindestens eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Die gerätegesteuerte Behandlung der mindestens einen Laborprobe entspricht bei einem Labor-Schüttelgerät einer Schüttelbehandlung, der die mindestens eine Probe unterzogen wird. Mögliche Parameter, insbesondere Programmparameter, insbesondere Nutzerparameter, die zur Beeinflussung einer Schüttelbehandlung verwendet werden, definieren insbesondere die Bewegungsintensität, insbesondere die Bewegungsfrequenz bei einem oszillierenden Antrieb, einer Zeitperiode bei der Schüttelbehandlung, und/oder mindestens einen Ablaufparameter, der den Ablauf, insbesondere die Reihenfolge, eines aus mehreren Schritten bestehenden Schüttelbehandlungsprogramms beeinflusst oder definiert.

Ein Labor-Mischer, auch bezeichnet als "mixing device", dient wie das Labor-Schüttelgerät der Bewegung einer Laborprobe, insbesondere zum Mischen einer mehrere Bestandteile aufweisenden Laborprobe. Im Vergleich zu einem Labor-Schüttelgerät ermöglicht ein Labor-Mischer Bewegungen mit höheren Frequenzen, insbesondere höheren Drehzahlen. Labor-Mischer, insbesondere deren Behandlungseinrichtung, können insbesondere zur Durchführung einer oszillierenden Bewegung eingerichtet sein. Labor-Mischer, insbesondere deren Behandlungseinrichtung, weisen insbesondere einen Antrieb zum Antreiben der Bewegung auf, weisen insbesondere eine Zeitgebereinrichtung auf, mit der zeitliche Parameter der Einstellung der Mischbehandlung steuerbar sind und weisen insbesondere mindestens eine Heiz-/Kühleinrichtung und mindestens eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Die gerätegesteuerte Behandlung der mindestens einen Laborprobe entspricht bei einem Labor-Mischer einer Mischbehandlung, der die mindestens eine Probe unterzogen wird. Mögliche Parameter, insbesondere Programmparameter, insbesondere Nutzerparameter, die zur Beeinflussung einer Mischbehandlung verwendet werden, definieren insbesondere die Bewegungsintensität, insbesondere die Bewegungsfrequenz bei einem oszillierenden Antrieb, einer Zeitperiode bei der Mischbehandlung, und/oder mindestens einen Ablaufparameter, der den Ablauf, insbesondere die Reihenfolge, eines aus mehreren Schritten bestehenden Mischbehandlungsprogramms beeinflusst oder definiert.

Ein Labor-Gefriergerät dient der Lagerung mindestens einer Laborprobe in einem Gefrierraum bei geregelten Temperaturen insbesondere im Tiefkühlbereich von -18° C bis -50 °C oder im Ultratiefkühlbereich von -50° C bis - 90° C. Ein Labor-Gefriergerät ist insbesondere kein Kühlschrank, der zum Kühlen bei Temperaturen insbesondere im Bereich von 0° C bis 10° C oder von - 10° C bis 10 °C verwendbar ist. Ein Labor-Gefriergerät, insbesondere die Behandlungseinrichtung des Labor-Gefriergeräts, weist insbesondere mindestens eine Kühleinrichtung und mindestens eine Regeleinrichtung mit mindestens einem Regelkreis auf, dem als Stellglied die mindestens eine Kühleinrichtung und als Messglied mindestens eine Temperaturmesseinrichtung zugeordnet ist. Ein Labor-Gefriergerät, insbesondere die Behandlungseinrichtung des Labor-Gefriergeräts, weist insbesondere ein Kontrollmessgerät zur Temperaturmessung und/oder insbesondere eine Alarmeinrichtung auf, mit der ein Alarmsignal ausgegeben wird, wenn die im Gefrierraum gemessene Temperatur einen erlaubten Temperaturbereich verlässt. Ein Labor-Gefriergerät, insbesondere die Behandlungseinrichtung des Labor-Gefriergeräts, kann insbesondere eine Informationsleseeinrichtung zum Lesen der Information aufweisen. Diese Information kann auf einem Informationsträger enthalten sein, der mit einem Artikel verbunden sein kann. Dieser Artikel kann insbesondere ein Probenbehälter sein, der mindestens eine Laborprobe enthalten kann. Der Informationsträger kann insbesondere einen RFID-Chip, oder andere Identifikationsmerkmale aufweisen, wie z.B. einen Barcode, einen Datamatrix Code, einen QR-Code, die mit geeigneten Verfahren lesbar sind. Die gerätegesteuerte Behandlung der mindestens einen Laborprobe entspricht bei einem Labor-Gefriergerät einer Tieftemperaturbehandlung, der die mindestens eine Probe unterzogen wird. Mögliche Parameter, insbesondere Programmparameter, insbesondere Nutzerparameter, die zur Beeinflussung einer Tieftemperaturbehandlung verwendet werden, definieren insbesondere die Temperatur des Gefrierraumsraums, in dem die mindestens eine Probe tiefgekühlt wird und/oder den Informationslesevorgang, der vorzugsweise durchgeführt wird, wenn ein mit einem Informationsträger versehener Artikel von einem Benutzer in das Labor-Gefriergerät überführt wird.

Ein Bioreaktor weist einen Behälter auf, in dem bestimmte Mikroorganismen, Zellen, Algen oder Pflanzen (z.B. Moose) unter möglichst optimalen Bedingungen kultiviert (auch: fermentiert) werden. Der Betrieb eines Bioreaktors ist somit eine Anwendung der Biotechnologie, die biologische Prozesse, insbesondere Biokonversion oder Biokatalyse, in technischen Einrichtungen nutzt bzw. nutzbar macht. Faktoren, die in den meisten Bioreaktoren insbesondere durch Einstellung entsprechender Parameter steuerbar oder kontrollierbar sind, sind die Zusammensetzung des Nährmediums, die Sauerstoffzufuhr, Temperatur, pH-Wert, Sterilität und/oder andere Faktoren. Zweck der Kultivierung in einem Bioreaktor kann die Gewinnung der Zellen oder von Bestandteilen der Zellen oder die Gewinnung von Stoffwechselprodukten sein. Diese können z. B. als Wirkstoff in der pharmazeutischen oder als Grundchemikalie in der chemischen Industrie verwendet werden. Auch der Abbau von chemischen Verbindungen kann in Bioreaktoren stattfinden, wie z. B. bei der Abwasserreinigung in Kläranlagen. Die Herstellung von Bier, Wein und anderen derartigen Produkten findet ebenfalls in Bioreaktoren statt. In Bioreaktoren werden unterschiedlichste Organismen für verschiedene Zwecke kultiviert. Ein Bioreaktor kann deshalb unterschiedlich ausgeführt sein. Er kann als Rührkesselreaktor ausgeführt sein, der ein Volumen von wenigen Millilitern bis hunderten Litern haben kann und mit Nährlösung gefüllt werden kann. Er kann auch als Festbettreaktor, Photobioreaktor verwendet werden bzw. ausgebildet sein. Ein Bioreaktor kann Teil eines Bioreaktor-Systems sein, vorzugsweise eines parallelen Bioreaktor-Systems. In einem solchen parallelen Bioreaktor-System wird eine Vielzahl von Bioreaktoren parallel betrieben und mit höherer Präzision kontrolliert. Ein Bioreaktor, insbesondere dessen Behandlungseinrichtung, weist insbesondere eine Rühreinrichtung auf zum Rühren der im Reaktorbehälter enthaltenen Probe, insbesondere des Nährmediums. Ein Bioreaktor, insbesondere dessen Behandlungseinrichtung, weist insbesondere eine Pumpeinrichtung zum Pumpen der vorzugsweise als Nährmedium ausgeführten Laborprobe auf. Ein Bioreaktor, insbesondere dessen Behandlungseinrichtung, weist insbesondere eine Einstelleinrichtung für die Einstellung eines Gasgehalts im Reaktorbehälter, insbesondere des Gehalts an CO₂ und/oder O₂ bzw. an Gelöstsauerstoff (DO) auf. Ein Bioreaktor, insbesondere dessen Behandlungseinrichtung, weist insbesondere eine Einstelleinrichtung für die Einstellung, insbesondere Regelung, eines pH-Werts in der Probe im Reaktorbehälter auf. Die gerätegesteuerte Behandlung der mindestens einen Laborprobe entspricht bei einem Bioreaktor insbesondere einer Nährmediumsbehandlung, der die mindestens eine vorzugsweise als Nährmedium ausgeführte Probe unterzogen wird. Mögliche Parameter, insbesondere Programmparameter, insbesondere Nutzerparameter, die zur Beeinflussung einer Nährmediumsbehandlung verwendet werden, definieren insbesondere die Temperatur des Nährmediums im Reaktorbehälter, und/oder die Geschwindigkeit der Rühreinrichtung, insbesondere Rotationsgeschwindigkeit, und/oder die Pumpgeschwindigkeit bzw. Dosiergeschwindigkeit, und/oder einen Gasgehalt im Nährmedium, insbesondere CO₂ und/oder O₂ bzw. Gelöstsauerstoff (DO), und/oder den pH-Wert des Nährmediums, und/oder mindestens einen Ablaufparameter, der den Ablauf, insbesondere die Reihenfolge, eines aus mehreren Schritten bestehenden Nährmediumsbehandlungsprogramms beeinflusst oder definiert.

Eine Sicherheitswerkbank dient insbesondere zur sicheren Lagerung oder Aufbewahrung von Gefahrenstoffen, insbesondere zur Erfüllung einer biologischen Schutzstufe. Diese Stufen sind insbesondere in der EU-Richtlinie 2000/54/EG über den Schutz der Arbeitnehmer gegen Gefährdung durch biologische Arbeitsstoffe bei der Arbeit normiert und in der Biostoffverordnung in Deutschland. Eine Sicherheitswerkbank soll verhindern, dass im Falle der Gefahrenentstehung bei in einer Sicherheitswerkbank gelagerten Laborproben die Umwelt gefährdet wird. Die Sicherheit wird insbesondere dadurch gewährleistet, dass die im Aufnahmebereich der Sicherheitswerkbank enthaltene Atmosphäre ausgetauscht und insbesondere gefiltert wird. Dabei wird insbesondere diese Atmosphäre von einer Fördereinrichtung durch den Aufnahmebereich gefördert und durch einen Filter bewegt, der die Atmosphäre filtert, insbesondere von Gefahrstoffen reinigt. Die Sicherheitswerkbank, insbesondere deren Behandlungseinrichtung, weist insbesondere eine Fördereinrichtung zum Befördern von Atmosphärengas auf, weist insbesondere eine Zeitgebereinrichtung zur Messung einer Filterbetriebsdauer und einer Lüfterbetriebsdauer auf und/oder weist insbesondere eine Messeinrichtung zur Messung einer geförderten Menge an Atmosphärengas auf. Die gerätegesteuerte Behandlung der mindestens einen Laborprobe entspricht bei einer Sicherheitswerkbank insbesondere einer Atmosphärengasbehandlung zur Behandlung des Atmosphärengases, in dem die mindestens eine Probe gelagert ist. Mögliche Parameter, insbesondere Programmparameter, insbesondere Nutzerparameter, die zur Beeinflussung einer Atmosphärengasbehandlung verwendet werden, definieren insbesondere die Temperatur des Atmosphärengases im Aufnahmebereich, und/oder die Strömungsgeschwindigkeit des von der Fördereinrichtung beförderten Atmosphärengases, die geförderte Luftmenge, die Filterbetriebsdauer, und/oder die Lüfterbetriebsdauer.

Ein Probenplatten-Lesegerät, auch bezeichnet als "plate reader" oder "microplate reader", ist ein Laborgerät zum Nachweis biologischer, chemischer oder physikalischer Ereignisse von Proben in Mikrotiterplatten. Sie sind weit verbreitet in der Forschung verwendet, Wirkstoffforschung, Bioassay Validierung, Qualitätskontrolle und Fertigungsprozesse in der pharmazeutischen und biotechnologischen Industrie und akademischen Organisationen. Das Probenplatten-Lesegerät kann insbesondere mindestens eine Lichtquelle oder Strahlungsquelle aufweisen, kann mindestens einen Photodetektor aufweisen, kann eine Temperaturkontrolleinrichtung zu Temperierung der Proben bzw. der Probenplatten aufweisen, kann einen Zeitgeber aufweisen. Probenreaktionen können in 6-1536 Well-Format Mikrotiterplatten getestet werden. Das häufigste Format für Probenplatten, insbesondere Mikrotiterplatten, das in akademischen Forschungslabors oder klinisch-diagnostische Laboratorien verwendet wird, ist eine 96-well plate (eine 8 mal 12 Matrix) mit einem typischen Einzelvolumen zwischen 100 und 200 µl pro Well. Mikrotiterplatten mit höherer Dichte (384 - oder 1536-Well-Mikroplatten) werden typischerweise für Screening-Anwendungen verwendet, wenn Durchsatz (Anzahl der pro Tag verarbeiteten Proben) und Assay-Kosten pro Probe zu kritischen Parametern werden, mit einem typischen Assay-Volumen zwischen 5 und 50 µl pro Well. Die Behandlung ist insbesondere eine optische Messung der Mikrotiterplatte, insbesondere die Messung einer Absorption, Fluoreszenz-Intensität, Lumineszenz, zeitaufgelöste Fluoreszenz, und/oder Fluoreszenzpolarisation. Mögliche Parameter, insbesondere Programmparameter, insbesondere Nutzerparameter, die zur Beeinflussung einer Messung verwendet werden, definieren z.B. die Intensität der Lichtquelle, die Sensitivität eines Photodetektors, eine Zeitdauer und/oder eine Temperatur.

Ein Laborautomat zur Behandlung von fluiden Proben, insbesondere Pipettierautomat, dient der programmgesteuerten Behandlung dieser Proben. Ein Laborautomat kann ein Laborgerät sein oder mindestens ein Laborgerät der vorgenannten Art aufweisen, und/oder kann zur Durchführung mindestens einer, mehrerer oder aller der von diesem vorgenannten Laborgerät ausführbaren Behandlungen ausgebildet sein. Ein Laborautomat weist die Behandlungseinrichtung zur automatischen, programmgesteuerten Behandlung der mindestens einen Laborprobe auf, wobei die Behandlung unter Verwendung von mehreren Programmparametern, die zumindest teilweise vom Benutzer gewählt werden, gesteuert wird. Dabei kann die Probe vom Laborautomaten, bzw. einer Behandlungseinrichtung des Laborautomaten, beispielsweise bewegt und/oder transportiert werden. Die Bewegung kann durch Transport in beweglichen Probenbehältern oder durch die Leitung in Schlauchsystemen, Kapillaren oder Pipettenspitzen erfolgen. Dabei werden flüssige Proben insbesondere durch Ansaugen, also Pipettieren, oder allgemeiner, durch Anlegen von Druckdifferenzen transportiert. Durch eine Behandlung der Probe kann eine Probe z.B. geteilt oder verdünnt werden. Die Inhaltsstoffe einer Probe können analysiert werden oder es können, z.B. durch eine chemische Reaktion, neue Inhaltsstoffe hergestellt werden, insbesondere unter Verwendung der Probe. Insbesondere im Zusammenhang mit der Bearbeitung und Analyse von DNA oder RNA oder deren Bestandteilen sind Laborautomaten hilfreich, um eine Fülle von Informationen innerhalb einer geeigneten Zeitspanne zu gewinnen oder viele solcher Proben zu analysieren. Diese Behandlungseinrichtung eines Laborautomaten weist meist eine Arbeitsfläche mit Arbeitsstationen auf, an denen Proben in verschiedener Weise bearbeitet oder gelagert werden können. Für den Transport von z.B. flüssigen Proben zwischen verschiedenen Positionen, insbesondere Probenbehältern, weist die Behandlungseinrichtung meist eine gerätegesteuerte Bewegungsvorrichtung und eine gerätegesteuerte Fluid-TransferEinrichtung auf, die z.B. ein Pipettiersystem aufweisen kann. Sowohl der Transport der Proben als auch deren Behandlung an den verschiedenen Stationen lässt sich gerätegesteuert durchführen, insbesondere programmgesteuert durchführen. Die Behandlung erfolgt dann vorzugsweise zumindest teilweise oder vollständig automatisiert.

Vorzugsweise kann der Benutzer des Laborautomaten die Art der Behandlung der Probe festlegen. Eine solche Behandlungsart kann insbesondere dienen:
der Nukleinsäureaufreinigung, insbesondere
   - "MagSep Blood gDNA": Aufreinigung von genomischer DNA aus Vollblut, insbesondere unter Verwendung des Eppendorf ® MagSep Blood gDNA Kits;
   - "MagSep Tissue gDNA": Aufreinigung von genomischer DNA aus lebendem Gewebe, insbesondere unter Verwendung des Eppendorf ® MagSep Tissue gDNA Kits;
   - "MagSep Viral DANN/RNA": Aufreinigung von viraler RNA oder DNA aus zellfreien Körperflüssigkeiten, insbesondere unter Verwendung des Eppendorf ® MagSep Viral DNA/RNA Kits;
und PCR-Anwendungen, insbesondere
   - "Compose Mastermix";
   - "Normalize Concentrations";
   - "Create Dilution Series";
   - "Setup Reactions".

Ein Laborgerät, insbesondere der Laborautomat, ist vorzugsweise so ausgebildet, dass die Steuerung der Behandlung der mindestens einen Laborprobe automatisch unter Verwendung der erfassten Programmparameter erfolgen kann. Ein Laborgerät, insbesondere der Laborautomat" insbesondere dessen Steuerungsprogramm ist vorzugsweise so ausgebildet, dass die vom Benutzer vorgenommenen Eingaben, insbesondere der mindestens eine Wert mindestens eines Programmparameters, dazu verwendet werden, um gegebenenfalls weitere erforderliche Programmparameter automatisch zu ermitteln, insbesondere durch Berechnung oder durch Vergleich mit Daten in einer Datenbank des Laborautomaten. Insbesondere werden vorzugsweise die zur Durchführung der Behandlung im Einzelnen vorzugsweise zu verwendenden Steuerparameter automatisch bestimmt. Durch diese Maßnahmen wird die Bedienung des Laborgerätes komfortabel, der Benutzer erspart sich insbesondere das Entwerfen eines Programmcodes, da diese Schritte insbesondere automatisch vom Laborgerät durchgeführt werden. In einer bevorzugten Ausgestaltung der Erfindung werden vom Benutzer nur die Eingaben abgefordert, die direkt mit der durchzuführenden Behandlung der Proben zu tun haben. Das sind häufig dieselben Angaben, die auch für eine manuelle Durchführung der Behandlung notwendig wären und dem Benutzer geläufig sind. Dagegen müssen solche Parameter, die die Steuerung des Laborgerätes betreffen, insbesondere die Steuerparameter, nicht im Einzelnen festgelegt werden, da diese vorzugsweise automatisch festgelegt werden. Steuerparameter sind die zur Steuerung der technischen Bestandteile der Behandlungseinrichtung im Einzelnen erforderlichen Parameter. Steuerparameter können Programmparameter sein oder können daraus für die technische Umsetzung abgeleitete Parameter sein, insbesondere automatisch bestimmte Parameter sein.

Vorzugsweise wählt ein Laborgerät, insbesondere der Laborautomat, ausgehend von der vom Benutzer gewählten Behandlungsart automatisch den passenden Satz von Programmparametern aus, dessen benutzerseitig erforderliche Programmparameter dann in den Schritten (b) und (c) vom Benutzer abgefragt wird. Der Programmparametersatz kann einerseits die benutzerseitig erforderlichen Programmparameter enthalten, und kann andererseits weitere Programmparameter enthalten. Diese weiteren Programmparameter können in Abhängigkeit von der gewählten Behandlungsart automatisch festgelegt werden, oder können in Abhängigkeit von mindestens einem, oder allen, vom Benutzer eingegebenen Programmparametern automatisch festgelegt werden, und/oder können in der Speichereinrichtung gespeichert sein. Die gespeicherten Parametersätze sind -oder werden vom Laborautomaten- vorzugsweise für die Behandlungsart optimiert, so dass der Benutzer vorzugsweise kein Spezialwissen zur Optimierung der Parameter benötigt. Aus dem Programmparametersatz werden die Steuerparameter abgeleitet, die zur Durchführung der konkreten Behandlung mittels der Behandlungseinrichtung notwendig sind.

Für eine Behandlungsart ist vorzugsweise ein Programmparametersatz von für diese Behandlungsart spezifischen Programmparametern definiert. Die Programmparameter dieses Programmparametersatzes können insbesondere die für die Behandlung zu verwendenden Zubehörteile, z.B. Probenbehältnis, Transportbehältnis, und/oder die zu verwendenden Tools, und/oder weiteres Verbrauchsmaterial definieren.

Die Zuordnung von Programmparametersatz und Behandlungsart ist in der Speichereinrichtung des Laborgerätes, insbesondere des Laborautomaten, gespeichert. Vorzugsweise ist der Laborautomat dazu ausgebildet, dass der Benutzer weiterer solcher Zuordnungen im Laborgerät speichern und/oder verwenden kann. Durch diese Zuordnung, in Kombination mit der übersichtlichen und gut strukturierten Abfrage der Programmparameter wird die Bedienung de Laborautomaten besonders effizient. Diese Zuordnung erfolgt vorzugsweise durch die Verwendung eines oder mehrere Programmmodule, wobei jeweils ein Programmmodul auf eine bestimmte Anwendung zugeschnitten ist:
Vorzugsweise weist der Laborautomat mindestens ein Programmmodul auf, wobei ein vorbestimmtes Programmmodul der Steuerung einer vorbestimmten Laboraufgabe zur Behandlung von Laborproben dient.

Vorzugsweise ist der mindestens eine Programmparameter, insbesondere der benutzerseitig erforderliche Programmparameter, aus der folgenden Menge von physikalischen Größen ausgewählt, die zur Behandlung einer Laborprobe mittels der Behandlungseinrichtung relevant sind: Probenanzahl, Verdünnungsfaktor, Zielvolumen, Position der Proben in einem Probengefäßhalter oder in einer Mikrotiterplatte, Probentemperatur, Zeitpunkte und/oder Zeitdifferenzen, Temperaturen oder Temperaturdifferenzen, Änderungsraten solcher Parameter, etc.

Vorzugsweise enthält das Steuerprogramm ferner Anweisungen, um folgenden Schritt auszuführen, insbesondere ist die Steuereinrichtung des Laborgeräts zur Durchführung des folgenden Schritts eingerichtet:
Erzeugen eines Methodenprogramms unter Verwendung der vom Benutzer eingegebenen Programmparameter, und Speichern des Methodenprogramms in der Speichereinrichtung, wobei das Methodenprogramm vom Benutzer editierbar ist. Dadurch wird die Verwendung des Laborgerätes, insbesondere des Laborautomaten, noch flexibler.

Der Laborautomat kann so verändert werden, dass damit weitere Behandlungsarten durchgeführt werden können. Dies kann dadurch geschehen, dass die dafür notwendigen Dateien und/oder Programme oder Programmbestandteile, insbesondere ein der Behandlungsart zugeordnetes Programmmodul, nachträglich in den Laborautomaten, insbesondere dessen Speichereinrichtung, übertragen werden.

Eine Laborprobe ist eine Probe, die in einem Labor behandelt werden kann. Anstelle des Begriffs Laborprobe wird bei der Beschreibung der Erfindung auch der Begriff "Probe" verwendet. Die Probe kann ein Fluid sein. Die Probe kann flüssig, gelartig, pulverförmig oder ein Festkörper sein, oder solche Phasen aufweisen. Die Probe kann ein Gemisch aus solchen Phasen sein, insbesondere ein Flüssigkeitsgemisch, eine Lösung, eine Suspension, z.B. eine Zellsuspension, eine Emulsion oder Dispersion. Eine Lösung ist ein homogenes Gemisch aus mindestens zwei Stoffen. Eine flüssige Probe kann eine solche sein, die üblicherweise in einem biologischen, chemischen, medizinischen Labor gehandhabt wird. Eine flüssige Probe kann eine Analysenprobe sein, ein Reagenz, ein Medium, ein Puffer etc. Eine Lösung weist einen oder mehrere gelöste feste, flüssige oder gasförmige Stoffe (Solute) auf, und weist ferner ein vorzugsweise flüssiges Lösungsmittel (Solvens) auf, das insbesondere den größeren Anteil oder größten Anteil des Volumens auf, das die Lösung bildet. Das Lösungsmittel kann selbst eine Lösung sein.

Die Behandlung einer Laborprobe(n) kann einen oder mehrere der nachfolgend genannten Vorgänge, insbesondere gleichzeitig oder nacheinander, beinhalten:
- Transport der Laborprobe, insbesondere durch eine Transporteinrichtung, unter Wirkung der Gravitation und/oder einer durch den Laborautomaten bewirkten Kraft;
- berührungslose (nicht-invasive) physikalische Behandlung der Probe, insbesondere thermische Behandlung, insbesondere Erwärmen und/oder Kühlen, insbesondere geregeltes Temperieren der Probe; oder Gefrieren oder Auftauen der Probe, oder sonstiges thermisches Herbeiführen einer Phasenänderung der Probe, z.B. Verdampfen, Kondensieren etc; magnetische Behandlung der Probe; optische Behandlung der Probe, insbesondere Bestrahlen der Probe mit Strahlung, insbesondere Licht, insbesondere sichtbare Licht. Infrarotlicht oder UV-Licht, oder Detektion von solcher Strahlung, insbesondere Fluoreszenzlicht, aus dieser Probe; magnetische Behandlung einer Probe mit magnetischen Bestandteilen, insbesondere magnetische Separation von magnetischen Bestandteilen, insbesondere "magnetic beads", von einer fluiden Phase der Probe; Bewegen der Probe, also Durchführen einer mechanischen Behandlung der Probe, insbesondere Schütteln, rotieren, oszillieren, vibrieren, zentrifugieren, akustische Behandlung, insbesondere mit Ultraschall, jeweils z.B. zum Zwecke des Mischens der Probe oder des Separierens von Bestandteilen innerhalb der Probe oder zum Transportieren der magnetischen Bestanteile aus der Probe heraus oder in die Probe hinein;
- invasive physikalische Behandlung der Probe, also Durchführen einer mechanischen Behandlung der Probe: Einbringen von Rührwerkzeug, z.B. Rührstab oder magnetischer Rührfisch in die Probe und Rühren, Einbringen einer Sonode für akustische oder Ultraschalbehandlung, Einbringen von Transportmitteln, insbesondere Transportbehältnissen in die Probe, z.B. Dispenserspitze oder Pipettenspitze, oder Hohlnadel oder Schlauch; Zugabe von anderen Hilfsmitteln in die Probe;
- chemische, biochemische oder biomedizinische Behandlung der Probe: Zugabe von chemischen (z.B. Reaktant, Reagenz, Solvens, Solut) biochemischen (z.B. biochemische Makromoleküle, z.B. DNA, DNA-Bestandteile; pharmazeutische Wirkstoffe), oder biomedizinischen (Blut, Serum, Zellmedium) Stoffen;
- Lagerung der Probe, insbesondere für einen programmgesteuert definierten Zeitraum, insbesondere unter bestimmten physikalische Bedingungen, z.B. bei bestimmter Temperatur, Temperaturen, oder Temperaturwechseln, insbesondere wiederholten Temperaturwechseln, z.B. zyklisch und/oder periodisch wiederholten Temperaturwechseln, und/oder Einstellen eines Umgebungsdruck, z.B. Anlegen eines Überdrucks oder eines Unterdrucks, insbesondere eines Vakuums, und/oder Einstellen einer definierten Umgebungsatmosphäre, z.B. ein Schutzgas oder eine bestimmte Luftfeuchtigkeit, unter bestimmten Strahlungsbedingungen, z.B. abgeschirmt gegen sichtbares Licht, im Dunkeln, oder unter definierter Bestrahlung;
- Messung oder Analyse der Probe, insbesondere Analyse durch eine nicht-invasive und/oder invasive Behandlung der Probe, insbesondere um mindestens eine oder mehrere chemische, physikalische, biochemische und/oder medizinische Eigenschaften der Probe zu messen; insbesondere Zählen von Zellen mittels Cell-Counter;
- Bearbeitung der Probe, insbesondere Änderungen mindestens einer Eigenschaft der Probe, insbesondere mittels nicht-invasiver und/oder invasiver Behandlung der Probe.

Diese Behandlung erfolgt insbesondere programmgesteuert, unter Verwendung mindestens eines Programmparameters.

Diese Behandlung erfolgt insbesondere gemäß mindestens einem Steuerparameter, der die Behandlung der Laborprobe mittels der Behandlungseinrichtung bestimmt. Ein Steuerparameter kann einen Zeitraum festlegen, einen Zeitpunkt, ein bestimmtes Probenvolumen und/oder Dosiervolumen, eine bestimmte Probentemperatur, etc. Ein Steuerparameter kann die automatische Verwendung eines bestimmten Transportkopfes, eines bestimmten Typs eines Transportbehältnisses, eines bestimmten Typs von Probebehältnissen, einer oder mehrerer individuelle Proben oder von bestimmten Positionen dieser Komponenten im Arbeitsbereich betreffen. Ein Steuerparameter kann die Behandlung einer individuellen Probe betreffen oder die Behandlung mehrerer oder vieler Proben.

Vorzugsweise wird ein Steuerparameter in Abhängigkeit von mindestens einem Programmparameter automatisch vom Laborgerät, insbesondere Laborautomat, ausgewählt, insbesondere automatisch in Abhängigkeit von den vom Benutzer ausgewählten Programmparametern automatisch ausgewählt. Dadurch hat der Benutzer den Vorteil, dass er nicht alle Steuerparameter einzeln bestimmen muss. Der Benutzer muss keine Kenntnisse über die Programmierung des Laborgeräts besitzen. Vielmehr erfolgt die Auswahl der für die Behandlung notwendigen Steuerparameter mittels der vom Benutzer eingegebenen Porgrammparameter. Dadurch ist die Verwendung des Laborgeräts besonders komfortabel.

Ein Steuerparameter kann auch einem Programmparameter entsprechen.

Der Transport einer Probe kann ein Transport aus einem Probenbehältnis in ein Transportbehältnis und/oder aus dem Transportbehältnis in ein Probenbehältnis oder einen sonstigen Zielort sein. Dieser Transport erfolgt insbesondere programmgesteuert, unter Verwendung mindestens eines Programmparameters.

Das Transportbehältnis kann z.B. ein Dispenserbehältnis sein, das einen beweglichen Kolben und eine Einlass-/Auslassöffnung aufweist. Der Kolben erzeugt einen Unterdruck bzw. Überdruck im Dispenserbehältnis und saugt so die Probe in das Behältnis hinein oder gibt sie wieder ab. Dieser Vorgang folgt dem Verdrängerprinzip, d.h. die zu bewegende, meist flüssige und somit inkompressibele Probe wird zwangsbewegt, indem das zuvor von der Probe eingenommene Volumen vom Kolben bewegt wird. Der Kolben wird in der Regel von einer Bewegungseinrichtung bewegt, insbesondere programmgesteuert bewegt, die dem Laborautomaten zugeordnet ist.

Das Transportbehältnis kann ferner eine Pipettenspitze sein. Eine Pipettenspitze weist eine Einlass-/Auslassöffnung auf, und eine zweite Öffnung. Die zweite Öffnung wird an eine Ansaugeinrichtung gekoppelt, so dass durch einen Unterdruck eine flüssige Probe aus einem Probenbehältnis in das Transportbehältnis gesaugt (pipettiert) werden kann. Die Abgabe der Probe erfolgt durch Ventilieren des Ansaugbereichs, mittels Gravitation und/oder über einen Überdruck, der z.B. über die zweite Öffnung im Pipettenspitze erzeugt wird.

Das Transportbehältnis besteht vorzugsweise teilweise oder vollständig aus Kunststoff. Es ist vorzugsweise ein Verbrauchsartikel, der typischer Weise nur für eine Behandlung oder eine geringe Zahl von Behandlungsschritten der Probe verwendet wird. Das Transportbehältnis kann aber auch teilweise oder vollständig aus einem anderen Material bestehen.

Der Transport einer Probe kann ein Transport der Probe von einer Ausgangsposition in eine Zielposition sein. Die Ausgangsposition kann vorliegen, wenn die Probe in einem ersten Probenbehältnis angeordnet ist und die Zielposition dieser Probe kann ihre Position in einem zweiten Probenbehältnis sein, in das die Probe übertragen wird. Diese Art des Transports wird vorliegend auch als Probentransfer oder Transfer bezeichnet. Ein Probentransfer wird in der Praxis meist durchgeführt, um eine Probe aus einem Vorratsbehältnis, in dem z.B. die Probe gelagert war und/oder das z.B. eine größere Menge der Probe enthalten kann, in ein zweites Probenbehältnis zu übertragen, in dem die Probe weiter behandelt wird. Dieser Transport erfolgt insbesondere programmgesteuert, unter Verwendung mindestens eines Programmparameters.

Das Transportbehältnis ist vorzugsweise mit einer Transporteinrichtung des Laborautomaten verbunden oder verbindbar.

Ein Probenbehältnis kann ein Einzelbehältnis sein, in dem nur eine einzige Probe enthalten ist, oder kann ein Mehrfachbehältnis sein, in dem mehrere Einzelbehältnisse miteinander verbunden angeordnet sind.

Ein Einzelbehältnis kann ein offenes Behältnis oder ein verschliessbares Behältnis sein. Bei einem verschliessbaren Behältnis kann ein Deckelelement, insbesondere eine Verschlusskappe, vorgesehen sein. Das Deckelelement kann fest mit dem Behältnis verbunden sein, z.B. als Klappdeckel oder Klappverschlusskappe, oder kann als separate Komponent verwendet werden.

Vorzugsweise sind bei einem Mehrfachbehältnis die mehreren Einzelbehältnisse in festen Positionen zueinander angeordnet, insbesondere entsprechend den Kreuzungspunkten eines Gittermusters angeordnet. Die vereinfacht die automatisierte Ansteuerung der Positionen und insbesondere die individuellen Adressierung von Proben. Ein Mehrfachbehältnis kann als Plattenelement ausgebildet sein, bei dem die Einzelbehältnisse so verbunden sind, dass sie eine plattenförmige Anordnung bilden. Die Einzelbehältnisse können als Vertiefungen in einer Platte ausgebildet sein oder können über Stegelemente miteinander verbunden sein. Das Plattenelement kann ein Rahmenelement aufweisen, in dem die Einzelbehältnisse gehalten werden. Diese Verbindungen von Komponenten können integrale Verbindungen sein, d.h. stoffschlüssige Verbindungen und/oder durch einen gemeinsamen Spritzgussprozess erzeugte Verbindungen sein, oder können kraftschlüssig (englisch "force-fit") und/oder formschlüssig (englisch "form-fit") erzeugt sein. Das Plattenelement kann insbesondere eine Mikrotiterplatte sein.

Mehrfachbehältnisse können eine Mehrzahl (von 2 bis 10) Einzelbehältnissen aufweisen. Sie können ferner eine Vielzahl (größer 10) aufweisen, typischer Weise 12, 16, 24, 32, 48, 64, 96, 384, 1536 Einzelbehältnisse. Das Mehrfachbehältnis kann insbesondere eine Mikrotiterplatte sein. Eine Mikrotiterplatte kann gemäß einem oder mehreren Industriestandard(s) ausgebildet sein, insbesondere der Industrienormen ANSI/SBS 1-2004, ANSI/SBS 2-2004, ANSI/SBS 3-2004, ANSI/SBS 4-2004.

Das maximale Probenvolumen, das von einem Transportbehältnis oder einem Probenbehältnis aufnehmbar ist, liegt typischer Weise zwischen 0,01 ml und 100 ml, insbesondere bei 10 - 100 µl, 100 - 500 µl, 0,5 - 5 ml, 5-25 ml, 25-50 ml, 50-100 ml, abhängig vom Typ des gewählten Transportbehältnis oder Probengefäßes.

Ein Probenbehältnis - gleichbedeutend verwendet zum Begriff Probengefäß- kann einen Informationsbereich aufweisen, der Informationen über das Probenbehältnis oder über dessen Inhalt aufweisen kann. Der Informationsbereich kann codierte Information aufweisen, z.B. einen Barcode oder QR-Code, oder einen RFID-Chip, oder eine anders codierte Information. Die Information kann Information zur Identifizierung der Probe und/oder eines Probenbehältnisses aufweisen. Der Laborautomat kann ein Informationsleseeinrichtung aufweisen, um diese Information zu lesen und, vorzugsweise der Steuereinrichtung zur Verfügung zu stellen.

Das Probenbehältnis besteht vorzugsweise teilweise oder vollständig aus Kunststoff. Es ist vorzugsweise ein Verbrauchsartikel, der typischer Weise nur für eine Behandlung oder eine geringe Zahl von Behandlungsschritten der Probe verwendet wird. Das Probenbehältnis kann aber auch teilweise oder vollständig aus einem anderen Material bestehen.

Das Probenbehältnis ist vorzugsweise mit einer Transporteinrichtung des Laborautomaten transportierbar.

Das Laborgerät, insbesondere der Laborautomat, ist vorzugsweise dazu ausgebildet, eine Vielzahl von Proben nacheinander und/oder parallel zu behandeln. Insbesondere ist ein Laborgerät, insbesondere der Laborautomat, vorzugsweise dazu ausgebildet, eine Vielzahl von Probengefäßen, insbesondere Einzelbehältnisse und/oder Mehrfachbehältnisse, programmgesteuert zu behandeln, insbesondere zu transportieren, zu entleeren und/oder zu befüllen.

Vorzugsweise weist ein Laborgerät, insbesondere der Laborautomat, genau einen Arbeitsbereich auf. Ein solches Laborgerät, insbesondere Laborautomat, ist kompakt und kann sich insbesondere zur Verwendung auf einem Labortisch eignen, wobei er dann insbesondere auch als Tischgerät bezeichnet wird. Der Tisch kann z.B. die Workbench eines chemischen, biochemischen oder biomedizinischen Labors sein. Das Laborgerät, insbesondere der Laborautomat, kann auch zur Aufstellung in einem solchen Labor ausgebildet sein. Ein Laborgerät, insbesondere Laborautomat, mit einem Arbeitsbereich kann ferner als unabhängig arbeitendes Gerät eines solchen Labors ausgebildet sein, oder kann in einen Geräteverbund eingebunden sein/werden.

Das Laborgerät, insbesondere der Laborautomat, kann auch als Laborstraße ausgebildet sein, bei dem mehrere Arbeitsbereiche so benachbart angeordnet sind, dass mittels eine Transportvorrichtung eine, mehrere oder eine Vielzahl von Proben, nacheinander und/oder parallel zwischen den Arbeitsbereichen transportiert werden können. Ein Arbeitsbereich einer Laborstraße ist vorzugsweise dazu ausgebildet, dass eine bestimmte Laboraufgabe, betreffend meistens die parallele und/oder sequentielle Behandlung einer Vielzahl von Proben, durchgeführt wird. Durch diese Spezialisierung jedes Arbeitsbereichs wird ein hoher Arbeitsdurchsatz der Laborstrasse erreicht. Zur Durchführung einer solchen bestimmten Aufgabe kann vorgesehen sein, dass in jedem Arbeitsbereich nur eine Art der Behandlung mindestens einer Probe, oder nur wenige Arten der Behandlung, z.B. zwei bis zehn Behandlungsarten durchgeführt werden. An jeder Arbeitsstation kann zur Durchführung einer Behandlung eine Behandlungseinrichtung angeordnet sein, die für ein bestimmtes Laborgerät, wie im Rahmen der Beschreibung der Erfindung beschrieben, charakteristisch ist. Die Transportvorrichtung kann ein Schienensystem aufweisen und/oder eine Robotereinrichtung zum programmgesteuerten Bewegen von Proben bzw. Probenbehältnissen.

Ein Laborgerät, insbesondere Laborautomat, kann mit einem LIMS verbunden oder verbindbar sein. LIMS steht für Labor-Informations- und Management-System. Ein LIMS ist in bekannter Weise ein Softwaresystem, das die Datenverarbeitung im chemischen, physikalischen, biologischen, medizinischen automatischen oder teilautomatischen Labor betrifft. Solche Daten können aus Messungen der Proben stammen, und/oder können die Steuerung der Bearbeitung der Daten betreffen. Ein LIMS dient vorzugsweise der Messwerterfassung und der Messwertauswertung. LIMS wird dazu verwendet, um den Arbeitsdurchsatz in einem Labor zu steigern und/oder die Effizienz der Behandlung von Laborproben zu optimieren.

Ein Werkzeugelement kann z.B. ein Transportkopf für den Fluidtransfer sein, insbesondere ein Pipettierkopf, der einen Verbindungsabschnitt zur Verbindung von einer Pipettenspitze (Einkanalpipettierkopf) oder zur Verbindung von mehreren Pipettenspitzen (Mehrkanalpipettierkopf) mit dem Pipettierkopf aufweisen kann. Über mindestens einen druck- und gasdichten Kanal, der mit dem Pipettierkopf verbunden ist, lässt sich Flüssigkeit in die mindestens eine Pipettenspitze saugen, wenn diese mit dem dem Verbindungsabschnitt verbunden ist. Dieses Pipettieren erfolgt beim Laborautomaten insbesondere programmgesteuert, insbesondere beeinflusst durch mindestens einen Programmparameter. Der Transportkopf kann auch ein Dispensierkopf sein, der mindestens eine Bewegungseinrichtung zur Bewegung eines Kolbens der Dispenserspitze aufweist. Die Bewegung der Bewegungseinrichtung erfolgt beim Laborautomaten insbesondere programmgesteuert, insbesondere beeinflusst durch mindestens einen Programmparameter. Der Transportkopf kann zur Flüssigkeitsdosierung dienen, insbesondere für die Dosierung in unterschiedlichen Bereichen; ein Transportkopf kann zur Dosierung einer flüssigen Probe mit einem Volumen ausgebildet sein, das aus einem für diesen Transportkopf spezifischen Volumenbereich ausgewählt sein kann: z.B. 1 - 50 µL, oder 20 - 300 µL,oder 50 -1000 µL, ("I" und "L" stehen jeweils als Abkürzung für Liter). Ein Transportkopf kann als Einkanalkopf ausgebildet sein, bei dem nur eine Probe transportiert wird, oder kann als Mehrkanal, insbesondere Achtkanal- oder 12-Kanal ausgebildet sein, bei dem mehrere Proben parallel bearbeitet bzw. transportiert werden. Es sind vorzugsweise spezifische Transportbehältnisse vorgesehen, die in Abhängigkeit vom jeweiligen Typ von Transportkopf, insbesondere gemäß dem entsprechenden Volumenbereich eingesetzt werden können.

Ein Werkzeugelement kann z.B. ein Transportkopf für den Transport von Gegenständen sein, z.B. eine Trage- und/oder Greifwerkzeug zum Tragen und /oder Greifen eines Gegenstands. Ein Tragewerkzeug kann einen Befestigungsabschnitt zum lösbaren Befestigen des Gegenstands am Tragewerkzeug aufweisen, z.B. durch kraftschlüssiges und/oder formschlüssiges und/oder magnetisches Verbinden des Gegenstands mit dem Tragewerkzeug. Auf diese Weise können innerhalb der Arbeitsfläche oder zwischen mehreren Arbeitsbereichen und/oder Arbeitsflächen.

Ein Werkzeugelement kann ferner eine Behandlungseinheit sein, z.B. zur Durchführung einer thermischen, akustischen, optischen und/oder mechanischen Behandlung mindestens einer Probe.

Der Laborautomat kann eine Informationsleseeinrichtung aufweisen, um eine Information über eine Probe und/oder ein Probenbehältnis und/oder eine Behandlungsanweisung für diese Probe und/oder dieses Probenbehältnis zu lesen und, vorzugsweise, der Steuereinrichtung des Laborautomaten zur Verfügung zu stellen.

Der Laborautomat weist vorzugsweise mindestens eine Zeitgebereinrichtung und/oder vorzugsweise eine Zeitnehmereinrichtung auf, um die zeitabhängige Behandlung der Proben zu ermöglichen. Die zeitabhängige Behandlung erfolgt vorzugsweise programmgesteuert, und insbesondere gesteuert durch mindestens einen Programmparameter.

In einer bevorzugten Gestaltung des erfindungsgemäßen Laborautomaten ist dieser dazu ausgebildet, in Abhängigkeit von der vom Benutzer gewählten Behandlungsart und der vom Benutzer eingegebenen Programmparameter automatisch eine oder mehrere der nachfolgend genannten Komponenten automatisch zur Verwendung bei der programmgesteuerten Behandlung auszuwählen:
- mindestens ein geeignetes Probenbehältnis, insbesondere geeignet zur Aufnahme mehrere Proben, die gemeinsam bearbeitet werden sollen, die z.B. gemischt werden sollen oder zwischen denen eine chemische Reaktion oder biochemische, biologische oder biomedizinische Wechselwirkung auftreten soll;
- mindestens ein geeignetes Transportbehältnis, insbesondere Pipettenspitze und/oder Dispenserspitze;
- mindestens einen geeigneten Transportkopf, mit dem das vorzugsweise automatisch gewählte Transportbehältnis verbindbar ist,
- mindestens ein geeignetes Werkzeugelement, das der Durchführung der gewünschten Behandlung dient.

Vorzugsweise ist der erfindungsgemäße Laborautomaten dazu ausgebildet, in Abhängigkeit von der vom Benutzer gewählten Behandlungsart und der vom Benutzer eingegebenen Programmparameter automatisch eine oder mehrere der nachfolgend genannten Steuerparameter automatisch zur Verwendung bei der programmgesteuerten Behandlung auszuwählen:
- mindestens ein Zeitraum, zu dem ein bestimmter Arbeitsschritt der Behandlung durchgeführt wird;
- mindestens ein Probenvolumen und/oder Dosiervolumen;
- mindestens eine Arbeitsposition der mindestens einen Arbeitsfläche;
- Bewegungsparameter zur Festlegung des für die gewünschte Behandlung der Probe erforderlichen Bewegungsablaufs der Robotereinrichtung des Laborautomaten.

Durch die automatische Auswahl der genannten Komponenten und/oder der Steuerparameter in Abhängigkeit von mindestens einem Programmparameter, insbesondere in Abhängigkeit von dem mindestens einen, vom Benutzer ausgewählten Programmparameter, ergibt sich für den Benutzer der Vorteil, dass er die Auswahl der Komponenten und Steuerparameter nicht selbst einzeln bestimmen muss. Vielmehr erfolgt die Auswahl der für die Behandlung notwendigen Steuerparameter mittels der vom Benutzer eingegebenen Programmparameter. Der Benutzer muss keine Kenntnisse über die Programmierung des Automaten besitzen. Dadurch ist die Verwendung des Laborautomaten besonders komfortabel.

Durch die automatische Auswahl der genannten Komponenten und/oder der Steuerparameter in Abhängigkeit von mindestens einem Programmparameter kann z.B. erreicht werden, dass aufgrund der Anwenderangaben (z.B. 20 Proben zu verdünnen) automatisch der richtige Pipettierkopf gegriffen wird, bzw., allgemeiner, der passende Tool, z.B. Transportkopf und/oder Werkzeugkopf verwendet wird. D.h. der Anwender muss dann nicht entscheiden, was das optimale Tool ist, sondern er entscheidet nur, was seine gewünschte Behandlung ist, z.B. die Nukleinsäureaufreinigung in einer gewünschten Weise. Der Benutzer, z.B. ein Biologe, eine biologisch-technischer Assistent, ein medizinisch-technischer Assistent muss dann also lediglich die Entscheidungen treffen, die er aufgrund seiner Ausbildung einfach und schnell treffen kann, er muss aber weder eine abstrakte Programmiersprache beherrschen, noch muss er größere Berechnungen anstellen.

Die Behandlungseinrichtung des Laborautomaten weist auf: vorzugsweise mindestens einen Arbeitsbereich, vorzugsweise mindestens eine Transporteinrichtung, vorzugsweise mindestens eine Behandlungseinheit.

Vorzugsweise hat das Laborgerät, insbesondere der Laborautomat, die Eigenschaft, die vom Benutzer eingegebenen Programmparameter dauerhaft zu speichern und später automatisch -oder vom Benutzer ausgelöst- wieder zu laden. Der Benutzer kann dann einzelne der Parameter ändern, um eine Probenbehandlungsart vollständig zu definieren. Dadurch wird der Bedienkomfort erhöht und die Fehleranfälligkeit verringert. Dies ist vor dem Hintergrund vorteilhaft, dass Laborgeräte besonders effizient für wiederkehrende Prozesse verwendet werden.

Das erfindungsgemäße Laborgerät weist vorzugsweise eine Kommunikationseinrichtung zur Herstellung einer Datenfernverbindung für den Datenaustausch mit einem externen Gerät auf, das ebenfalls eine geeignete Kommunikationseinrichtung zur Herstellung einer Fernverbindung für den Datenaustausch mit dem Laborautomat aufweist.

Das Laborgerät weist vorzugsweise eine Benutzerschnittstelleneinrichtung zur manuellen Eingabe von Daten durch einen Benutzer auf, und zur Anzeige von Informationen, insbesondere von in diesen Daten enthaltenen Informationen, wobei die Benutzerschnittstelleneinrichtung eine Anzeigeneinrichtung, insbesondere ein Display, insbesondere ein Touch-Screen-Display aufweist.

Das erfindungsgemäße Laborgerät kann mehrere Behandlungseinrichtungen aufweisen. Eine Benutzerschnittstelleneinrichtung kann mehreren erfindungsgemäßen Laborgeräten zugeordnet bzw. zuordenbar sein, insbesondere mit diesen über eine zweite Schnittstelleneinrichtung und insbesondere zweite Datenverbindungen verbindbar sein oder verbunden sein. Dadurch kann mit einer Benutzerschnittstelleneinrichtung der Zugriff der Benutzer auf mehr als ein Laborgerät oder auf ein Laborgerät mit mehr als einer Behandlungseinrichtung ermöglicht sein.

Die Erfindung betrifft ferner ein Verfahren zur Erfassung von mindestens einer Benutzereingabe an einem erfindungsgemäßen Laborgerät, das gemäß mindestens einem der vorherigen Ansprüche gestaltet ist, aufweisend die Schritte:
- Erfassung mindestens einer Benutzerbewegung am Anzeigebereich mittels der Sensoreinrichtung;
- Festlegen mindestens eines Programmparameters und/oder dessen Wert in Abhängigkeit von der mindestens einen Benutzerbewegung,
- Anzeigen eines grafischen Skizzenelements im Anzeigebereich, das die mindestens eine Benutzerbewegung repräsentiert.

Weitere mögliche bevorzugte Ausgestaltungen des erfindungsgemäßen Verfahrens lassen sich aus der Beschreibung des erfindungsgemäßen Laborgeräts und von dessen bevorzugten Ausgestaltungen ableiten.

Weitere bevorzugte Ausgestaltungen des erfindungsgemäßen Laborgeräts und des erfindungsgemäßen Verfahrens ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1 zeigt schematisch ein Ausführungsbeispiel des erfindungsgemäßen Laborgeräts in einer isometrischen perspektivischen Ansicht.
Fig. 2a zeigt den Anzeigebereich eines erfindungsgemäßen Laborgeräts in einer ersten Gestaltung zur Bereitstellung eines bewegungserfassenden Eingabemodus.
Fig. 2b zeigt den Anzeigebereich eines erfindungsgemäßen Laborgeräts in einer ersten Gestaltung zur Bereitstellung eines bewegungserfassenden Eingabemodus.
Fig. 2c zeigt den Anzeigebereich eines erfindungsgemäßen Laborgeräts in einer dritten Gestaltung zur Bereitstellung eines bewegungserfassenden Eingabemodus.
Fig. 2d zeigt den Anzeigebereich eines erfindungsgemäßen Laborgeräts in einer vierten Gestaltung zur Bereitstellung eines bewegungserfassenden Eingabemodus.
Fig. 2e zeigt den Anzeigebereich eines erfindungsgemäßen Laborgeräts in einer fünften Gestaltung zur Bereitstellung eines bewegungserfassenden Eingabemodus.
Fig. 2f zeigt den Anzeigebereich eines erfindungsgemäßen Laborgeräts in einer sechsten Gestaltung zur Bereitstellung eines bewegungserfassenden Eingabemodus.
Fig. 2g zeigt den Anzeigebereich eines erfindungsgemäßen Laborgeräts in einer siebten Gestaltung zur Bereitstellung eines bewegungserfassenden Eingabemodus.
Fig. 2h zeigt den Anzeigebereich eines erfindungsgemäßen Laborgeräts in einer achten Gestaltung zur Bereitstellung eines bewegungserfassenden Eingabemodus.
Fig. 3 zeigt ein weiteres Ausführungsbeispiel des erfindungsgemäßen Laborgeräts, einen Thermocycler.

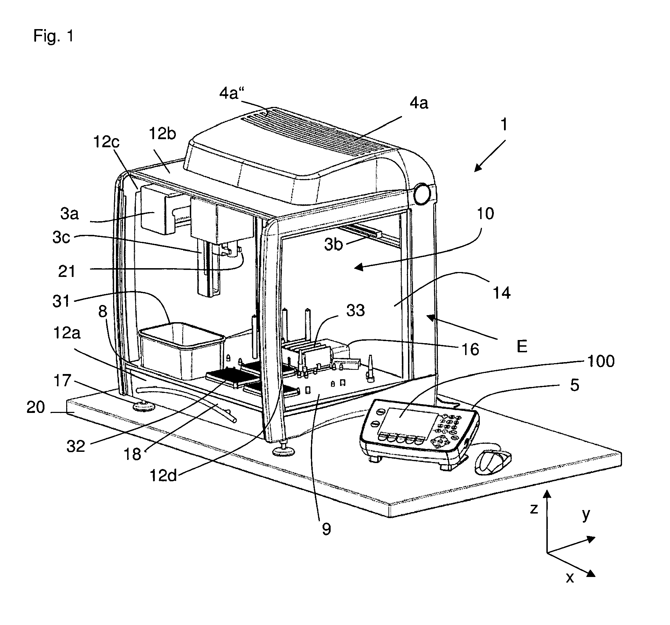

Fig. 1 zeigt das Laborgerät 1, das hier als Laborautomat 1 zur Behandlung von fluiden Proben, und zwar als Pipettierautomat ausgebildet ist, der insbesondere einen integrierten Thermocycler (nicht gezeigt) aufweist. Der Laborautomat 1 dient der programmgesteuerten Behandlung dieser Proben.

Fig. 1 zeigt den Laborautomat 1 für die automatisierte Verarbeitung von flüssigen Proben, insbesondere für die programmgesteuerte Behandlung von flüssigen Proben. Der Laborautomat 1 ist ein Tischgerät und ist mit seinen vier Sockeln 17 auf dem Arbeitstisch 20 angeordnet. Es verfügt über eine elektronische Steuereinrichtung 2 (nicht gezeigt), die geeignet ist, einen Programmcode für die programmgesteuerte Behandlung der flüssigen Proben zu verarbeiten. Die Steuereinrichtung 2 ist in dem Steuerraum angebracht, der durch den Pfeil E bezeichnet ist und der von dem Arbeitsraum 10 durch eine vertikale Wand 14 getrennt ist. Der Steuerraum beherbergt auch die Spannungsversorgungskomponenten, die die geeignete Versorgungsspannung für die elektrischen Komponenten des Laborautomaten liefern.

Der Laborautomat 1 weist einen Behandlungsraum 10 zur Aufnahme der flüssigen zu behandelnden Proben auf, eine programmgesteuert steuerbare Probenbearbeitungseinrichtung 3, zum Durchführen von mindestens einem programmgesteuerten Behandlungsschritt an der mindestens einen Probe, die in dem Bearbeitungsraum angeordnet ist. Der Probenbearbeitungseinrichtung 3 sind die Komponenten 3a, 3b, 3c und 3d der Bewegungseinrichtung zugeordnet.

Der Laborautomat 1 weist ein Gehäuse 12 auf, das eine Vorderseite 12a aufweist, eine gegenüber der Vorderseite angeordnete Rückseite 12f (nicht gezeigt), eine Oberseite 12b, eine gegenüber der Oberseite angeordnete Unterseite 12e (nicht gezeigt) und gegenüberliegenden laterale Seiten 12c und 12d. Die Seiten 12a, 12b und 12c sind im Wesentlichen aus einem Material, das für sichtbares Licht transparent ist.

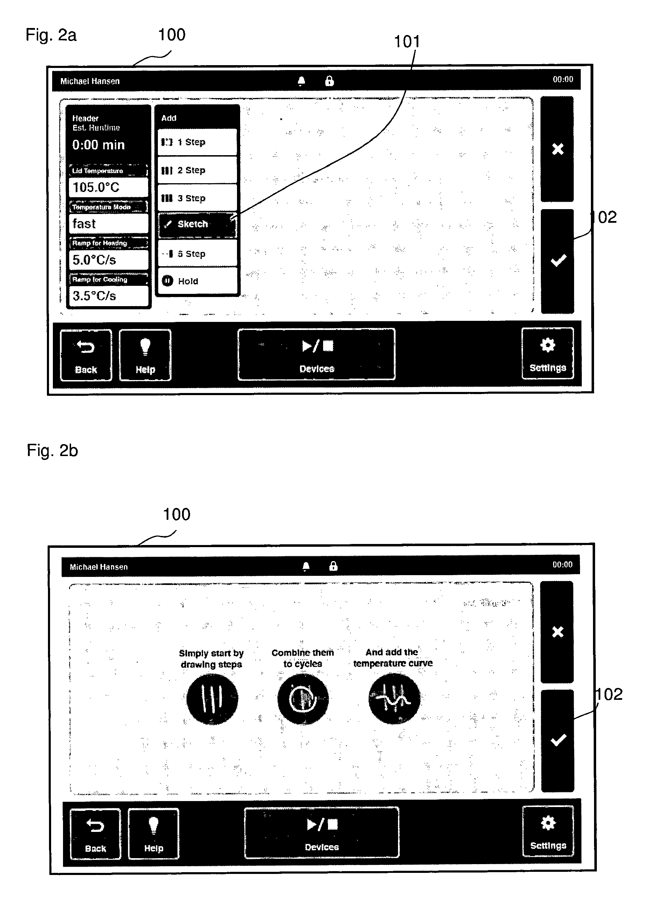

Die Vorderseite 12a, die im Wesentlichen wie eine Tür 12a, nämlich eine Schiebetür 12a ausgebildet ist, kann von Hand bewegt werden und/oder programmgesteuert bewegt werden und kann und sich nach unten im Wesentlichen entlang der z- Achse des kartesischen Koordinatensystems schließen. In Fig. 2a ist die geschlossene Position der Tür 12a gezeigt.

Der Behandlungsraum 10 ist durch die Vorderseite 12a und die beiden Seitenflächen 12c und 12d sowie die Wand 14 und die Arbeitsfläche 8, welche die obere Seite der Bodenplatte 9 bildet, beschränkt. Die Arbeitsfläche 8 stellt sechs Bearbeitungsstationen zur Verfügung. Die Bearbeitungsstationen sind im Wesentlichen ebenen Flächen im Bearbeitungsbereich 8. Stifte dienen dazu, Lab-Ware, also beispielsweise Thermorack 33, Mikrotiterplatten 32 und Abfallbehälter 31 an der jeweiligen Bearbeitungsstation auszurichten. Die genaue Positionierung ermöglicht eine präzise robotergesteuerte Adressierung der Probenbehälter, insbesondere der Vertiefungen in den Mikrotiterplatten 32. Eine magnetische Trennvorrichtung 16 ist in der Nähe der Wand 14 angeordnet, wo ein Thermorack 33, d.h. eine temperaturgesteuerte Probengefäßhalterung, angeordnet ist. Die magnetische Gabel (nicht gezeigt) der magnetischen Trennvorrichtung 16 fährt von der Seite in entsprechende Aufnahmekanäle des Thermoracks ein, um seitlich an den Laborgefäßen (Probenröhrchen) ihre magnetische Wirkung zu entfalten.

Der Laborautomat 1 weist zwei Dekontaminationseinrichtungen auf, eine elektronisch steuerbare Luftreinigungsvorrichtung für die Reinigung der Luft im Behandlungsraum, die elektronisch und digital durch die Steuereinrichtung gesteuert wird und die eine Entlüftungsvorrichtung 4a, 4a" aufweist. Die Belüftungsvorrichtung weist drei Ventilatoren auf (nicht dargestellt), die einen Luftstrom von außerhalb der Vorrichtung in den Behandlungsraum transportieren.

Die Steuereinrichtung 2 weist ein Steuerprogramm auf. Der Laborautomat 1 weist eine Probenbearbeitungseinrichtung 3 auf, die eine Bewegungseinrichtung aufweist, mit drei Schienenelementen 3a, 3b, 3c, die Bewegungen entlang der y, x und z- Achse des kartesischen Koordinatensystems entsprechen. Zum Antrieb der Bewegung entlang der gewünschten Richtung sind elektronisch regelbare Linearmotoren vorgesehen. Auf diese Weise kann der Montagekopf 21 in jede gewünschte Position zugänglich in den Bearbeitungsraum 10 bewegt werden. Die Bewegungseinrichtung ist Teil eines Robotersystems der Probenbearbeitungseinrichtung 3. Mit dieser ist der Montagekopf 21 programmgesteuert transportierbar. Mit dem Montagekopf ist ein Werkzeuggerät verbindbar, z. B. ein Pipettierkopf oder ein Greifer. Die im Behandlungsraum angeordneten Bauteile, insbesondere die Probenbearbeitungseinrichtung 3, sind Bestandteil der Behandlungsvorrichtung des Laborautomaten.

Der Laborautomat weist eine Benutzerschnittstelleneinrichtung 5 auf, mit der ein Benutzer Eingaben am Laborautomaten vornehmen kann. Die Benutzerschnittstelleneinrichtung 5 weist mindestens ein Display auf, hier genau ein Display, das als Touchscreen ausgebildet ist mit einer Darstellungsgenauigkeit zwischen 100 dpi und 350 dpi und einer Ortsauflösung der Einzelsensoren der Sensoreinrichtung des Touchscreens, die gleich oder geringer sein kann im Vergleich zu der Darstellungsauflösung des Touchscreens. Im Touchscreen befindet sich mindestens ein Anzeigebereich, vorliegend genau ein Anzeigebereich 100, an dem der Benutzer durch Berühren des Bildschirms an einzelnen Punkten oder Bereichen oder mittels Durchführung einer Kurvenbewegung am Bildschirm eine Eingabe durchführen kann.

Die vorliegende Erfindung nutzt in einem vorteilhaften bewegungserfassenden Eingabemodus des Laborgeräts eine Benutzerbewegung, mittels der ein linienartiges grafisches Skizzenelement am Display erzeugt wird.

In Fig. 2a bis 2h ist jeweils eine im Anzeigebereich 100 des Laborgeräts anzeigbare Bedienoberfläche gezeigt. Diese bezieht sich auf die Programmierung des in das Laborgerät 1 integrierten Thermocyclers, dessen geregelt temperierbarer Probenaufnahmeblock (Temperierblock) an der Arbeitsstation mit dem Bezugszeichen 33 integriert ist (Fig. 1).

Die in Fig. 2a gezeigte Bedienoberfläche ermöglicht beim Thermocycler die Planung des Temperierprozesses für die im Temperierblock angeordneten (bzw. noch zu transportierenden) Proben. Bei diesem Temperierprozess werden zur Vervielfältigung von z.B. DNA-Abschnitten in einer PCR-Probe mehrere für bestimmte Zeiten anzulegende Temperaturstufen zyklisch wiederholt, in jedem Zyklus verdoppelt sich idealerweise die Anzahl der DNA-Abschnitte in der Probe. Mindestens zwei Temperaturstufen sind pro Zyklus erforderlich. Der Wechsel zwischen den Temperaturstufen erfolgt in festgelegten Geschwindigkeiten, auch bezeichnet als "ramps". Diese Geschwindigkeiten, ebenso wie andere Programmparameter, können auf einen Standardwert voreingestellt sein, der vom Benutzer änderbar sein kann, oder von einem Administrator, oder aber nur herstellerseitig. Der bewegungserfassende Eingabemodus des erfindungsgemäßen Laborgeräts wird über die Berührung des als virtuelle Eingabetaste gestalteten Teilbereichs 101 des Anzeigebereichs 100 aktiviert, auch bezeichnet als "Schaltfläche 101". In einer ebenfalls bevorzugten Ausgestaltung ist die "Schaltfläche 101" nicht vorgesehen und der bewegungserfassende Eingabemodus während der Anzeige dieser Benutzeroberfläche automatisch aktiviert.

Nach Aktivieren der Schaltfläche 101 wechselt die Bedienoberfläche z.B. zunächst zu einer Darstellung, wie in Fig. 2b gezeigt, in der Erläuterungen bzw. Hilfsinformationen zum bewegungserfassenden Bedienmodus dargestellt sind, wobei der Bedienmodus vorliegend derart einfach gestaltet ist, dass den Benutzern in der Regel dessen Verwendung ohne gesondertes Gestentrainingsverfahren gelingt. Es wird erläutert, dass zur Eingabe drei verschiedene Arten von charakteristischen Benutzerbewegungen (Gesten) zur Eingabe möglich sind, nämlich horizontale Bewegungen, mittels derer Temperaturstufen betreffende Programmparameter definiert werden und dazu senkrechte Bewegungen, mittels derer Zeitwerte betreffende Programmparameter definiert werden, ferner kreisförmige Auswahlbewegungen, mittels derer mehrere Grafikobjekte zur Definition des Inhalts eines Temperierzyklus ausgewählt werden.

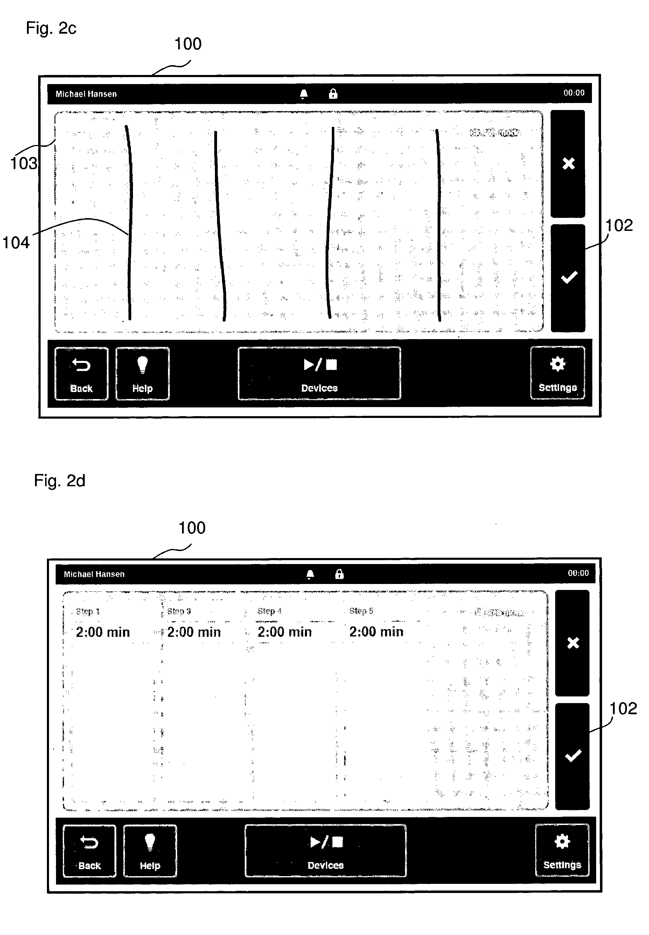

Nach Bestätigung mittels Berührung der Schaltfläche 102 wird von der Steuereinrichtung die Anzeige der in Fig. 2c gezeigten Bedienoberfläche bewirkt. Dort wird ein Darstellungsbereich 103 angezeigt, der im bewegungserfassenden Eingabemodus als Zeichenoberfläche benutzbar ist, auf der der Benutzer Skizzen zeichnen kann, die den Gesten entsprechen. Wie noch erläutert wird, wird die Erstreckung des Darstellungsbereich 103 in horizontaler Richtung entlang einer virtuellen Achse (x-Achse) als "Zeitstrang" dazu genutzt, um die Eingabe von Zeitwerten zu ermöglichen. Die Erstreckung des Darstellungsbereich 103 in zur horizontalen Richtung senkrechten Richtung entlang einer weiteren virtuellen Achse (y-Achse) als "Temperaturachse" dazu genutzt, um die Eingabe von Temperaturwerten zu ermöglichen.

Andere Bewegungen, die von der Steuereinrichtung nicht als eine der möglichen Gesten erkannt werden, führen zur Anzeige einer Meldung an den Benutzer. Im Darstellungsbereich 103 sind vier vertikale lineare Skizzenelemente (vertikale Striche) angezeigt. Diese wurden zuvor von einem Benutzer an genau der Stelle auf dem Anzeigebereich des Display erstellt, indem jeweils eine lineare vertikale den Anzeigenbereich entsprechend berührende Benutzerbewegung erkannt wurde und im wesentlichen ohne Zeitverzögerung angezeigt wurde, so dass sich für den Benutzer ein intuitives Schaffenserlebnis einstellte.

Nach Bestätigung mittels Berührung der Schaltfläche 102 wird von der Steuereinrichtung die Anzeige der in Fig. 2d gezeigten Bedienoberfläche bewirkt. Dort ist grafisch illustriert, wie die Steuereinrichtung die vier vertikalen Gesten des Benutzers zur Definition von Programmparametern bildenden Zeitwerten genutzt hat. Die erste Leistung des Laborgeräts im bewegungserfassenden Eingabemodus liegt darin, dass die "vertikale" Ausrichtung der Benutzerbewegung dazu genutzt wurde, um die Eingabe auf den Programmparameter "Zeitwert" zu beziehen. Bei einer horizontalen Benutzerbewegung wäre die horizontale Ausrichtung dazu genutzt worden, um die Eingabe auf den Programmparameter "Temperaturwert" zu beziehen. Zudem wurde die Anzahl von insgesamt vier nebeneinander durchgeführten vertikalen Strichgesten dahingehend interpretiert, dass insgesamt vier Temperaturstufen zeitlich hintereinander geplant werden sollen, wobei der standardmäßige Zeitperiode zur Durchführung der Temperierung auf einer Temperaturstufe bei 2 Minuten liegt. Der Übergang zwischen den Temperaturstufen erfolgt standardmäßig mittels der beschriebenen Rampen. Der hier definierte Programmparameter betrifft somit die Anzahl der hintereinander bei einer noch zu bestimmenden Temperatur durchzuführenden Temperierschritte. Indirekt betrifft der Programmparameter somit eine Zeitachse bei der Definition des zu planenden PCR Prozesses. Als Alternative Eingabeform wäre eine Zeitachse denkbar, auf der der Benutzer mittels senkrechter Strichgesten den Start bzw. das Ende der Temperierschritte einträgt, z.B. erfassbar in zeitlichen Inkrementen von 15 Sekunden. Ein Temperierschritt ist vorliegend als einer von vier nebeneinander liegenden rechteckförmigen Teilbereichen 105 dargestellt, die dem Darstellungsbereich der rechteckigen Zeichenebene 103 überlagert sind. Standardmäßig wurde hier eine Zeitperiode von 2 Minuten verwendet.

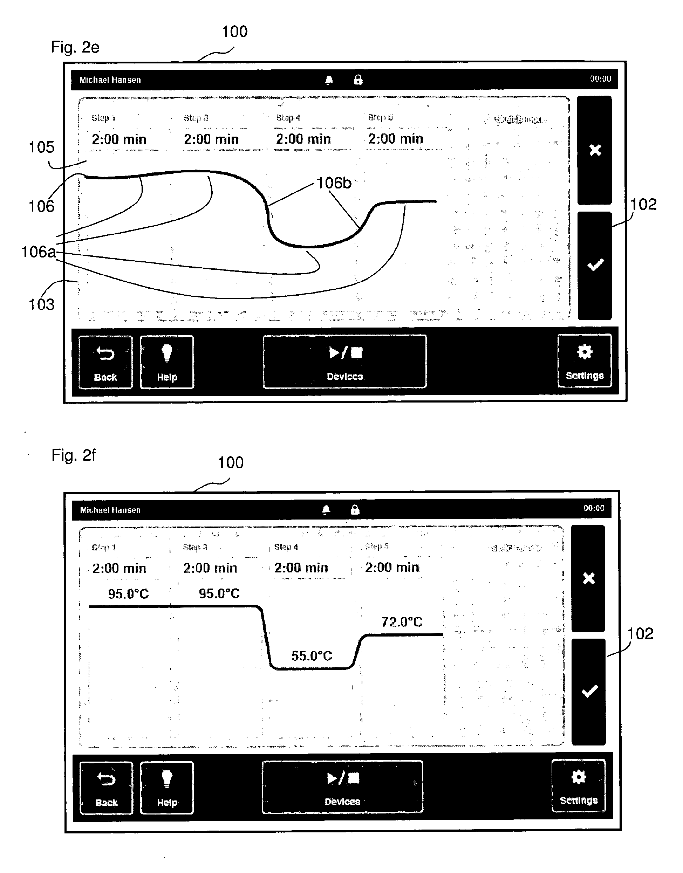

Die Bedienoberfläche mit dem Darstellungsbereich 103 aus Fig. 2d wird vom Benutzer als neue Zeichenebene verwendet. Der Benutzer zeichnet darauf die Kurve 106, die in Fig. 2e gezeigt ist. Der Benutzer führt auf der rechteckigen Zeichenebene 103 eine kontinuierliche Kurvenbewegung durch, die mehrere horizontale Abschnitte 106a aufweist und gebogene Abschnitte 106b beinhaltet, welche diese horizontalen Abschnitte verbinden. Im Eingabemodus wird innerhalb eines Zeitbereichs, der einem Teilbereich 105 entspricht, automatisch der auf dem Teilbereiche 105 gezeichnete horizontale Abschnitt 106a zur Definition der in diesem Zeitbereich zu applizierenden. Temperatur herangezogen. In Fig. 2f ist gezeigt, wie die Steuereinrichtung aus der Kurve 106 die auf die nacheinander folgenden Zeitabschnitte 105 bezogenen Informationen entnommen hat, die zur Anzeige der Temperaturen 95°C, 95°C, 55°C und 72°C in der idealisierten Soll-Temperaturkurve 106' geführt hat, die in Fig. 2f im Darstellungsbereich 103 angezeigt ist. Die y-Achse des Darstellungsbereichs ist virtuell in zunächst drei vertikal übereinander liegende rechteckige Bereiche strukturiert; ein horizontaler Kurvenabschnitt 106a im obersten Abschnitt wird automatisch der Temperatur 95°C zugeordnet, ein horizontaler Kurvenabschnitt 106a im mittleren Abschnitt wird automatisch der Temperatur 72°C zugeordnet, ein horizontaler Kurvenabschnitt 106a im untersten Abschnitt wird automatisch der Temperatur 55°C zugeordnet. Diese drei Temperaturwerte sind Standardwerte, die der Benutzer nachträglich fein justieren kann. Dazu kann der Benutzer einen horizontalen Kurvenabschnitt in Fig. 2e nachträglich berühren und den damit assoziierten Temperaturwert (Programmparameter dieses Temperaturabschnitts) ändern. Das kann z.B. technisch so umgesetzt sein, dass der Benutzer den horizontalen Kurvenabschnitt verschieben kann, wobei ihm der dann geänderte assoziierte Temperaturwert "life" numerisch angezeigt wird, bei Erreichen des gewünschten Temperaturwerts lässt der Benutzer die Kurve los, er beendet die entsprechende Bewegung. Aus der Kurve 106 werden so Paare von Werten gewonnen, bestehend aus Zeitabschnittnummer (bzw. Zeitintervall) und Temperaturstufe.

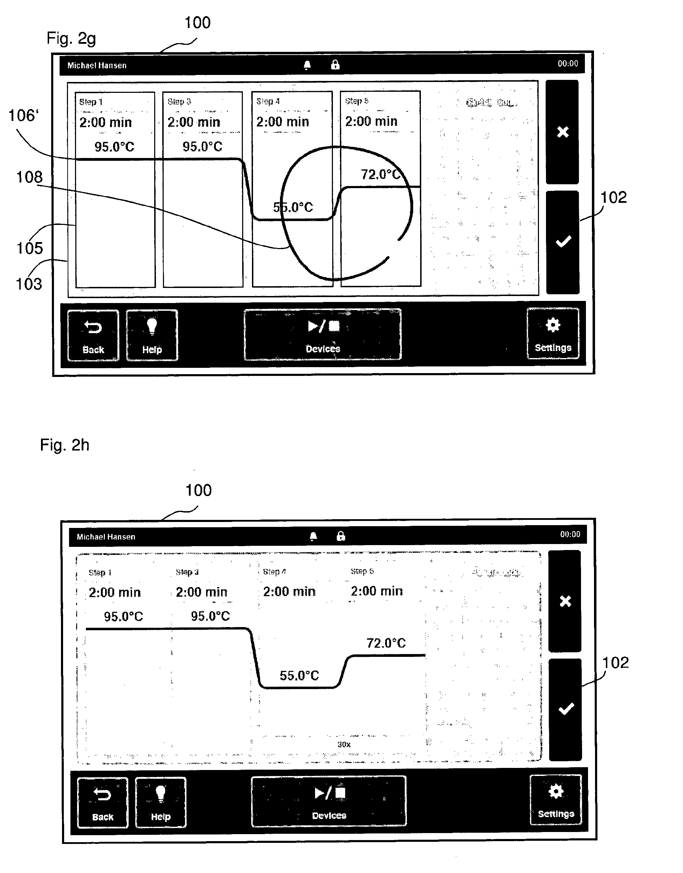

In Fig. 2g ist gezeigt, wie die Steuereinrichtung mittels einer im wesentlichen kreisförmigen Auswahlbewegung des Benutzers, dargestellt als im wesentlichen kreisförmiges Skizzenelement 108, mehrere Paare von Werten, bestehend aus Zeitabschnittnummer und Temperaturstufe, zur Definition eines PCR-Zyklus vorsieht. Der Benutzer markiert einfach durch den Kreis vorliegend zwei Teilbereiche 105 des Darstellungsbereichs 103 im Anzeigebereich 100. Dadurch werden die mit diesem Programmbereich assoziierten Programmparameter ausgewählt und gehen in den nächsten Programmschritt ein, in dem vom Benutzer die Anzahl A der gewünschten Wiederholungen des Zyklus abgefragt wird.

In Fig. 2h ist der fertig geplante PCR-Prozess gezeigt, bei dem grafisch hervorgehoben ist, dass der zuletzt definierte Zyklus mit zwei Wertepaaren aus (Zeitintervall, Temperaturstufe) A=30 mal wiederholt werden soll.

Die so entwickelte Eingabemöglichkeit führt zu einer anwenderfreundlichen, intuitiven Bedienung des Laborgeräts und eines effizienten, fehlerarmen Workflows.

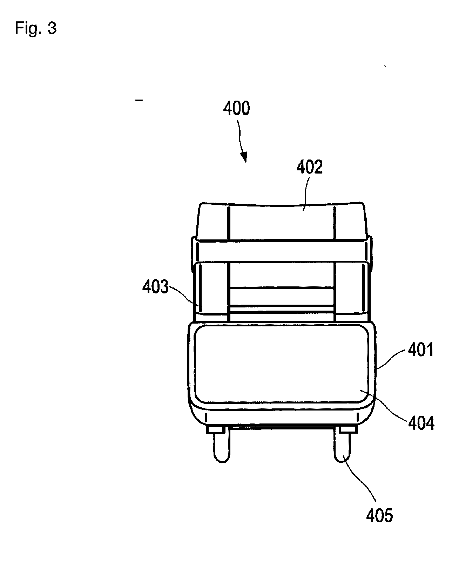

Fig. 3 zeigt ein Laborgerät 400, ein Thermoycler, ausgebildet für die automatisierte Verarbeitung von flüssigen Proben, insbesondere für die programmgesteuerte Temperierung von flüssigen Proben. Das Laborgerät 400 ist ein Tischgerät. Es verfügt über eine integrierte, elektronische Steuereinrichtung 406 (nicht gezeigt), die geeignet ist einen Programmcode für die programmgesteuerte Behandlung der flüssigen Proben zu verarbeiten. Die Steuereinrichtung 406 ist in dem Gehäuse 401 untergebracht. Das Gehäuse beherbergt auch die Spannungsversorgungskomponenten, die die geeignete Versorgungsspannung für die elektrischen Komponenten des Thermocyclers liefern.

Das Laborgerät 400 weist einen Behandlungsraum 403 zur Aufnahme der flüssigen zu behandelnden Proben auf, der mindestens eine programmgesteuert steuerbare Behandlungseinrichtung 408 (nicht gezeigt) aufnehmen kann, zum Durchführen von mindestens einem programmgesteuerten Behandlungsschritt an der mindestens einen Probe, die in der Behandlungseinrichtung angeordnet ist, welche in dem Bearbeitungsraum angeordnet ist. Der Behandlungsraum kann mit einem Deckel 402 verschlossen werden, um eine definierte Temperierungsumgebung herzustellen. In Fig. 4 ist das Laborgerät in geschlossenem Zustand dargestellt. Die Steuereinrichtung 406 weist ein Steuerprogramm auf.

Das Laborgerät weist eine Benutzerschnittstelleneinrichtung, nämlich einen Touchscreen 404 auf zur manuellen Eingabe von Daten durch einen Benutzer, und zur Anzeige von Informationen, die insbesondere von diesen Daten abhängig sein können, wobei die Benutzerschnittstelleneinrichtung ein Display aufweist, auf dem ein Anzeigebereich darstellbar ist. Der Touchscreen 404 weist eine bewegungserfassende Sensoreinrichtung auf (nicht sichtbar), die zur Erfassung mindestens einer Benutzerbewegung eingerichtet ist, die von einem Benutzer an dem Anzeigebereich durchführbar ist. Die Steuereinrichtung ist zur Bereitstellung eines bewegungserfassenden Eingabemodus eingerichtet ist, um • in Abhängigkeit von der mindestens einen Benutzerbewegung den mindestens einen Programmparameter zu wählen und/oder dessen Wert festzulegen, und • in Abhängigkeit von der mindestens einen Benutzerbewegung in dem Anzeigebereich mindestens ein grafisches Skizzenelement anzuzeigen, dass die mindestens eine Benutzerbewegung repräsentiert. Der Thermocycler 404 ist insbesondere zur Verwirklichung der in den Figuren 2a bis 2h gezeigten Ausgestaltungen ausgebildet, insbesondere zur Realisierung eines bewegungserfassenden Eingabemodus, wie er anhand der Figuren 2a bis 2h beschrieben ist.

## Patentansprüche

1. Laborgerät (1) zur gerätegesteuerten Behandlung mindestens einer Laborprobe, das aufweist:
mindestens eine Behandlungseinrichtung zur programmgesteuerten Behandlung der mindestens einen Laborprobe, wobei die Behandlung unter Verwendung von mehreren Programmparametern, die zumindest teilweise als Benutzerparameter vom Benutzer bestimmt werden, vom Laborgerät gesteuert wird,
eine Steuereinrichtung, aufweisend mindestens eine Prozessoreinrichtung zur Datenverarbeitung, wobei diese Datenverarbeitung die Ausführung eines Steuerprogramms zum Steuern des Laborautomaten beinhaltet, und aufweisend mindestens einer Speichereinrichtung zum Speichern von Daten, insbesondere dem Steuerprogramm und den Programmparametern,
eine Benutzerschnittstelleneinrichtung (5) zur manuellen Eingabe von Daten durch einen Benutzer, und zur Anzeige von Informationen, die insbesondere von diesen Daten abhängig sein können,
wobei die Benutzerschnittstelleneinrichtung ein Display aufweist, auf dem ein Anzeigebereich (100) darstellbar ist, und
wobei die Benutzerschnittstelleneinrichtung eine bewegungserfassende Sensoreinrichtung aufweist, die zur Erfassung mindestens einer Benutzerbewegung eingerichtet ist, die von einem Benutzer an dem Anzeigebereich durchführbar ist, und
wobei die Steuereinrichtung zur Bereitstellung eines bewegungserfassenden Eingabemodus eingerichtet ist, um
• in Abhängigkeit von der mindestens einen Benutzerbewegung den mindestens einen Programmparameter zu wählen und/oder dessen Wert festzulegen, und
• in Abhängigkeit von der mindestens einen Benutzerbewegung in dem Anzeigebereich mindestens ein grafisches Skizzenelement anzuzeigen, dass die mindestens eine Benutzerbewegung repräsentiert.

2. Laborgerät gemäß Anspruch 1, **dadurch gekennzeichnet, dass** die Steuereinrichtung zur Bereitstellung eines bewegungserfassenden Eingabemodus eingerichtet ist, um im Anzeigebereich entlang einer ersten linearen Achse mögliche Werte mindestens eines ersten Programmparameters, z.B. eines Zeitwerts, anzuzeigen und in diesem Eingabemodus eine Benutzerbewegung senkrecht zu der einen ersten linearen Achse zu erfassen, um den mit dieser Achse assoziierten Programmparameters auszuwählen und/oder dessen Wert zu bestimmen.

3. Laborgerät gemäß Anspruch 1 oder 2, **dadurch gekennzeichnet, dass** die Steuereinrichtung zur Bereitstellung eines bewegungserfassenden Eingabemodus eingerichtet ist, um im Anzeigebereich entlang einer zweiten linearen Achse mögliche Werte mindestens eines zweiten Programmparameters, z.B. einer Temperatur, anzuzeigen, und in diesem Eingabemodus eine Benutzerbewegung senkrecht zu der einen zweiten linearen Achse zu erfassen, um den mit dieser Achse assoziierten Programmparameter auszuwählen und/oder dessen Wert zu bestimmen.

4. Laborgerät gemäß Anspruch 2 oder 3, **dadurch gekennzeichnet, dass** die Steuereinrichtung in dem bewegungserfassenden Eingabemodus dazu eingerichtet ist, eine Position der linearen, senkrechten Benutzerbewegung entlang der linearen Achse zu erfassen, wobei diese Position für diesen Wert charakteristisch ist.

5. Laborgerät gemäß einem der Ansprüche 2 bis 4, **dadurch gekennzeichnet, dass** die Steuereinrichtung dazu eingerichtet ist, die Eingabe eines Wertes durch die Erfassung der Position der Benutzerbewegung senkrecht zur linearen Achse inkrementell vorzusehen.

6. Laborgerät gemäß einem der vorherigen Ansprüche, **dadurch gekennzeichnet, dass** die Steuereinrichtung dazu eingerichtet ist, um in dem bewegungserfassenden Eingabemodus durch die Benutzerbewegung Paare von Programmparametern oder Paare von Werten eines Programmparameters oder von zwei Programmparametern zu erfassen, die durch eine Punkteabfolge oder eine Kurve an zwei zueinander senkrechten Achsen im Anzeigenbereich repräsentiert werden, wobei diese Punkteabfolge oder diese Kurve das grafische Skizzenelement bilden.

7. Laborgerät gemäß einem der vorherigen Ansprüche, **dadurch gekennzeichnet, dass** die Steuereinrichtung dazu eingerichtet ist, während des bewegungserfassenden Eingabemodus im Anzeigenbereich mehrere grafische Objekte darzustellen, von denen jedes einen Programmparameter oder einen Wert eines Programmparameters repräsentiert, und eine im wesentlichen kreisförmige Benutzerbewegung, die mindestens eines -vorzugsweise mehrere- dieser grafischen Objekte berührt und/oder umrahmt, als Auswahlbewegung zu verwenden, mittels der die mehreren Programmparameter oder deren Werte ausgewählt werden.

8. Laborgerät gemäß einem der vorherigen Ansprüche, **dadurch gekennzeichnet, dass** die Benutzerschnittstelleneinrichtung mindestens ein weiteres Eingabemittel aufweist, und dass die Steuereinrichtung dazu eingerichtet ist, eine weitere Änderung des mindestens einen Programmparameters oder dessen Wertes mittels des mindestens einen weiteren Eingabemittels zu ermöglichen, nachdem dieser mindestens eine Programmparameter oder dessen Wert durch die Benutzerbewegung gewählt und/oder festgelegt wurde.

9. Laborgerät gemäß einem der vorherigen Ansprüche, das ein Thermocycler ist.

10. Laborgerät gemäß einem der vorherigen Ansprüche, das ein Pipettierautomat ist.

11. Pipettierautomat gemäß Anspruch 10, wobei die Steuereinrichtung dazu eingerichtet ist, eine Eingabe mittels Benutzerbewegung in der Weise zu verwenden, um manuell ein Pipettiermuster zu definieren.

12. Pipettierautomat gemäß Anspruch 10 oder 11, wobei die Steuereinrichtung dazu eingerichtet ist, die Eingabe einer Prozessplanung für einen mehrere Arbeitsstationen des Pipettierautomaten benötigenden Prozess zu ermöglichen, nämlich im Anzeigebereich einen Arbeitsbereich des Pipettierautomaten grafisch zu repräsentieren, in dem mehrere Arbeitsstationen grafisch dargestellt sind, wobei ein erster im Anzeigenbereich dargestellter Teilbereich eine erste Arbeitsstation, ein zweiter Teilbereich eine zweite Arbeitsstation, ein n-ter Teilbereich eine n-te Arbeitsstation (n=1...N, N eine natürliche Zahl) repräsentiert, wobei mit einem -insbesondere jedem- Teilbereich mindestens ein Programmparameter oder ein bestimmter Wert eines Programmparameters assoziiert ist, und eine Benutzerbewegung zu erfassen, deren Darstellung als grafisches Skizzenelement auf dem Anzeigenbereich mindestens einen oder mindestens zwei Teilbereiche berührt oder umrahmt, und dadurch mindestens einen oder mindestens zwei Programmparameter oder dessen Wert auswählt, der mit dem Teilbereich assoziiert ist.

13. Verfahren zur Erfassung von mindestens einer Benutzereingabe in einem Eingabemodus eines Laborgeräts, das gemäß mindestens einem der vorherigen Ansprüche gestaltet ist, aufweisend die Schritte:
- Erfassung mindestens einer Benutzerbewegung am Anzeigebereich mittels der bewegungserfassenden Sensoreinrichtung;
- Festlegen mindestens eines Programmparameters und/oder dessen Wert in Abhängigkeit von der mindestens einen Benutzerbewegung,
- Anzeigen eines grafischen Skizzenelements im Anzeigebereich, das die mindestens eine Benutzerbewegung repräsentiert.

14. Verfahren nach Anspruch 13, wobei durch die Eingaben des Eingabemodus ein zeitabhängiger Prozessablauf definiert wird, insbesondere ein automatisiertes PCR-Verfahren oder die zeitliche Abfolge von Probenbehandlungen an Arbeitsstationen eines Laborgeräts.
